# FASCICULE DE BREVET EUROPEEN

(11) **EP 0 425 378 B1**
(45) Date de publication et mention de la délivrance du brevet: **19.10.1994**
(21) Numéro de dépôt: 90403003.8
(22) Date de dépôt: 25.10.1990
(51) Int. Cl.: C07H 19/04, C07D 205/08

(54) **Nouveaux dérivés de l'azétidin-2-one, 1,3,4-trisubstitues, intermédiaires utiles pour la synthèse des bêta-lactames, et leur procédé de synthèse asymétrique**
1,3,4-Trisubstituierte Azetidin-2-on-Derivate, zur beta-Lactam-Synthese nützliche Zwischenprodukte und deren Verfahren zur asymmetrischen Herstellung
1,3,4-Trisubstituted azetidin-2-one derivatives, useful intermediates for synthesis of beta-lactames, and their process of asymmetrical preparation

(30) Priorité: 25.10.1989 FR 8913969
(43) Date de publication de la demande: 02.05.1991
(73) Titulaire: ADIR ET COMPAGNIE, 92415 Courbevoie Cédex (FR)
(72) Inventeur: Gateau-Olesker, Alice, F-91190 Gif sur Yvette (FR); Gero, Stéphane, F-91400 Les Ulis (FR); Cleophax, Jeannine, F-91120 Palaiseau (FR); Mercier, Daniel, F-91190 Gif sur Yvette (FR)

(56) Documents cités:
- US-A- 4 384 998
- JOURNAL OF ANTIBIOTICS, vol. 37, no. 10, octobre 1984, pages 1103-1129, Tokyo,JP; R. LABIA et al.: "Stereospecific constructions of chiral beta-lactams"
- TETRAHEDRON LETTERS, vol. 26, no. 1, 1985, pages 33-36, Pergamon Press, Oxford,GB; A.K. BOSE et al.: "Enantiospecific synthesis of beta-lactams viacycloaddition"
- THE JOURNAL OF ORGANIC CHEMISTRY, vol. 53, no. 18, 2 septembre 1988, pages4227-4236, American Chemical Society; D.R. WAGLE et al.: "Studies on lactams.81. Enatiospecific synthesis and absolute configuration of substituted beta-lactams from D-glyceraldehyde acetonide"

## Description

La présente invention concerne des nouveaux dérivés de l'azétidin-2-one, 1,3,4-trisubstitués, et leur procédé de synthèse asymétrique.

Les bêta-lactames constituent la classe la plus importante d'agents antibactériens connus jusqu'à présent. Il est également connu que les dérivés chiraux de l'azétidin-2-one sont des intermédiaires utiles pour la synthèse asymétrique des antibiotiques bêta-lactamiques. Plusieurs procédés de synthèse énantiosélective sont décrits dans la littérature. Certains parmi eux utilisent comme intermédiaires des bases de Schiff chirales. Différents inducteurs d'asymétrie ont été proposés tels que la D-thréonine (A.K. Bose et al., Tetrahedron Lett.,(1985),26,p.33-36), et le D-glyceraldéhyde, (D.R. Wagle et al.; J.Org.Chem.,(1988),53,p.4227-4236).

Les composés de l'invention sont des intermédiaires très utiles et intéressants pour la préparation des monobactames 3,4-disubstitués. En effet, les composés de la présente invention sont préparés avec un très bon rendement et de manière stéréosélective, à partir de matières premières peu coûteuses. Ils sont issus d'un procédé de synthèse qui utilise la D-glucosamine comme inducteur d'asymétrie, matière première facilement accessible. Ce procédé ne nécessite aucune étape onéreuse de résolution.

De plus, compte-tenu de leur structure chimique, les composés de l'invention permettent l'application d'une méthode efficace et originale pour cliver l'auxiliaire chiral en une seule étape et obtenir quantitativement des monobactames 3,4-disubstitués et non substitués au niveau de l'azote en position 1. Ces derniers composés constituent des intermédiaires fondamentaux dans la synthèse d'antibiotiques antibactériens.

La présente invention concerne plus spécialement des composés de formules générales I_{A} et I_{B}, qui constituent la formule générale I: 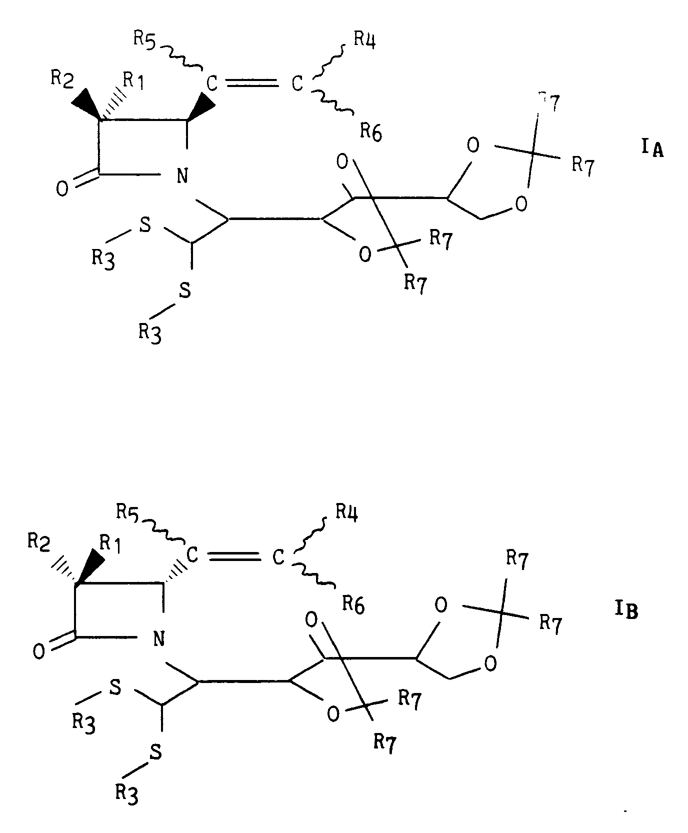
dans lesquelles :
R₁ représente un atome d'hydrogène, un atome d'halogène, un radical alkyle de 1 à 6 atomes de carbone, un radical trifluorométhyle, un radical alcoxy de 1 à 6 atomes de carbone, un radical phényle, phénoxy ou benzyle (éventuellement substitués sur le cycle benzénique par un ou plusieurs atomes d'halogène, des radicaux alcoxy de 1 à 6 atomes de carbone ou des radicaux alkyle de 1 à 6 atomes de carbone), un radical benzyloxy, un radical cyano, un radical alcoxycarbonyle de 2 à 7 atomes de carbone, un radical carboxy ou un radical alkylamino de 1 à 6 atomes de carbone (à condition toutefois que dans ce cas R₂ ne représente jamais simultanément un atome d'hydrogène),
R₂ différent de R₁, représente un atome d'hydrogène, un atome d'halogène, un radical alkyle de 1 à 6 atomes de carbone, un radical trifluorométhyle, un radical alcoxy de 1 à 6 atomes de carbone, un radical phényle, phénoxy ou benzyle (éventuellement substitués sur le cycle benzénique par un ou plusieurs atomes d'halogène, des radicaux alcoxy de 1 à 6 atomes de carbone ou des radicaux alkyle de 1 à 6 atomes de carbone), un radical benzyloxy, un radical cyano, un radical alcoxycarbonyle de 2 à 7 atomes de carbone, un radical carboxy ou un radical alkylamino de 1 à 6 atomes de carbone (à condition toutefois que dans ce cas R₁ ne représente jamais simultanément un atome d'hydrogène),
chaque R₃ représente un radical alkyle de 1 à 6 atomes de carbone ou les deux R₃ forment ensemble et avec les atomes de la fonction dithioacétal auxquels ils sont attachés un cycle de 5 à 7 chaînons,
R₄ représente un radical phényle (éventuellement substitué par un ou plusieurs atomes d'halogène, par un ou plusieurs radicaux alkyle de 1 à 6 atomes de carbone, par un radical alcoxy de 1 à 6 atomes de carbone), un radical naphtyle, ou un radical furyle,
R₅ et R₆ identiques ou différents représentent chacun un atome d'hydrogène, un radical alkyle de 1 à 7 atomes de carbone, ou un radical aryle (éventuellement substitué par un ou plusieurs atomes d'halogène, des radicaux alcoxy de 1 à 6 atomes de carbone ou des radicaux alkyle de 1 à 6 atomes de carbone),
et
chaque R₇ représente un radical méthyle ou les deux R₇ forment ensemble et avec l'atome de carbone auquel ils sont attachés un noyau cyclohexane ou un noyau cyclopentane.

La présente invention concerne aussi un procédé de préparation des composés de formule générale I,
caractérisé en ce que l'on condense la D-glucosamine composé de formule II : 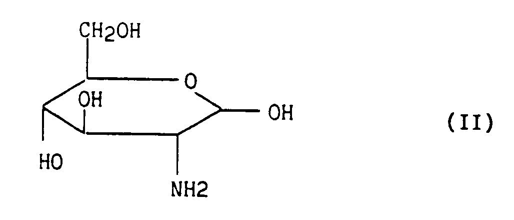
. soit avec une quantité équimolaire d'un composé de formule III_{A} :

   **HS(CH₂)**_{**n**}**SH III**_{**A**}

   dans laquelle n est égal à 2-4,
. soit avec une quantité au moins double d'un composé de formule III_{B} :

   **HSR'₃ III**_{**B**}

   dans laquelle R'₃ représente un radical alkyle de 1 à 6 atomes de carbone, en présence d'acide chlorhydrique concentré,
   pour obtenir un dérivé de D-glucosamine de formule IV: 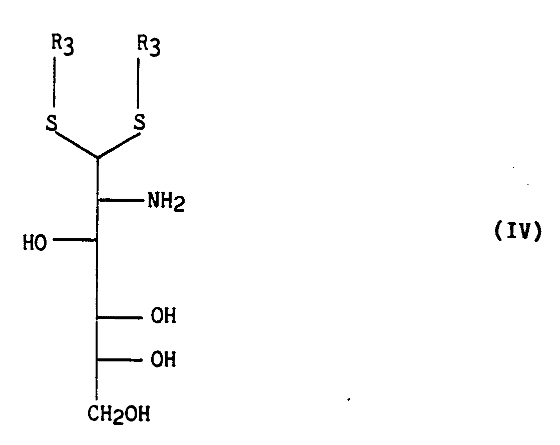 dans laquelle R₃ a la même signification que pour la formule I,
   sur lequel on fait réagir un composé de formule V : 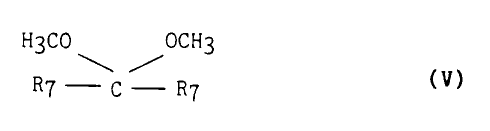 dans laquelle la signification de R₇ reste identique à celle donnée pour la formule I, en présence de l'acide paratoluènesulfonique,
   pour obtenir un dérivé de formule VI : 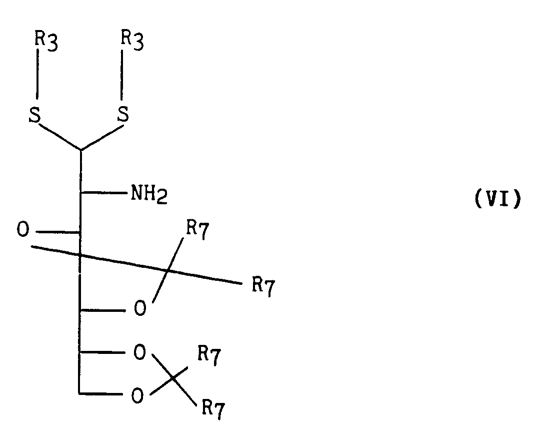 dans laquelle R₃ et R₇ ont la même signification que pour la formule I,
   que l'on condense avec un aldéhyde de formule VII : 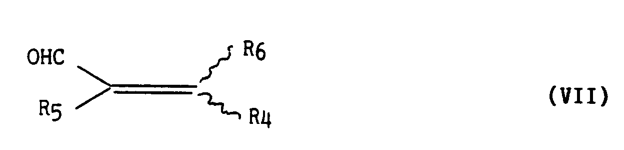 dans laquelle la signification de R₄, R₅ et R₆ est identique à celle donnée pour la formule I,
   pour obtenir une base de Schiff de formule VIII : 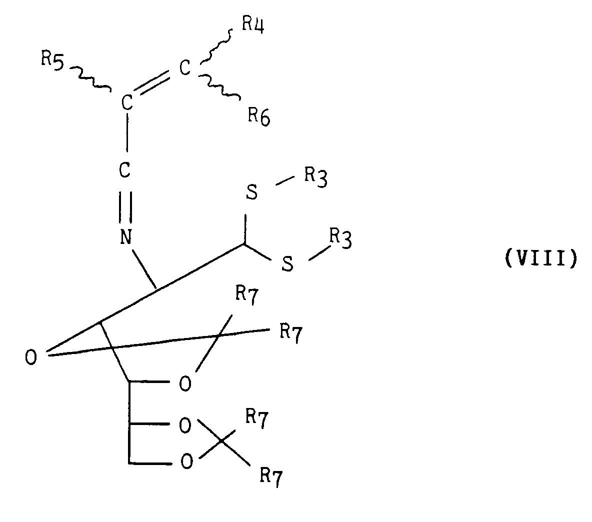 dans laquelle R₃, R₄, R₅, R₆ et R₇ ont la même signification que pour la formule I,
   que l'on cyclise ensuite en présence de triéthylamine avec un chlorure d'acide de formule IX: 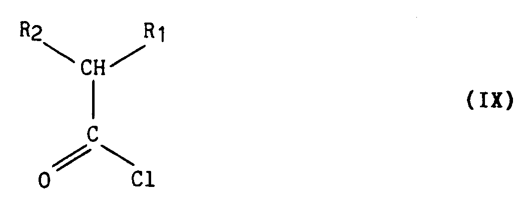 dans laquelle R₁ et R₂ ont la même signification que pour la formule I,
   pour obtenir un mélange de monobactames de formule générale I_{A} et I_{B}, que l'on sépare ensuite par cristallisation et/ou par chromatographie préparative.

A partir des composés I_{A} et I_{B}, grâce à leur nature et à une méthode de clivage de l'auxiliaire chiral, efficace et originale, on obtient facilement les monobactames X_{A} et X_{B} : 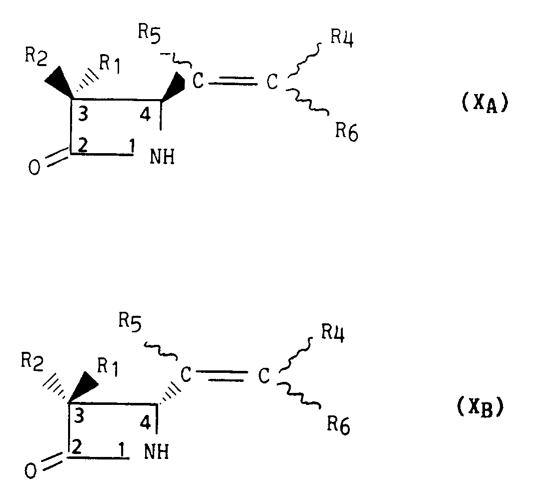
Pour les formules X_{A} et X_{B}, la signification de R₁, R₂, R₄, R₅ et R₆ reste identique à celle donnée pour les formules I_{A} et I_{B}.

En effet, en soumettant les composés de formule I_{A} à l'action du n-butyllithium, ou d'un autre réactif chimique équivalent, on obtient, de manière presque quantitative, les composés de formule X_{A} et respectivement, à partir des composés I_{B}, les composés de formule X_{B}.

Cette méthode originale de clivage fait aussi partie de la présente invention.

Le procédé décrit permet l'obtention des composés I_{A} et I_{B} avec un très bon rendement. En effet, il s'agit d'une cycloaddition de "type STAUDINGER" d'un cétène et d'une imine, pour obtenir des azétidinones 1,3,4-trisubstituées.

L'avantage de la présente invention réside dans le fait que les diastéréoisomères I_{A} et I_{B} préparés à partir de la D-glucosamine sont obtenus et séparés facilement. En outre, les intermédiaires préparés lors des différentes étapes sont obtenus avec des rendements en général supérieurs à 90%. Les composés chiraux de formule IV sont aisément obtenus sous forme cristalline, par traitement de la D-glucosamine, en présence d'acide chlorhydrique par un thiol (composé de formule III_{B}) ou un dithiol (composé de formule III_{A}).

Pour la préparation des composés de formule IV, la quantité nécessaire de la D-glucosamine et de dithiol est équimolaire. Par contre, lors de la réaction de la D-glucosamine avec un thiol, la quantité nécessaire de ce dernier composé pour la réaction est deux fois molaire. Le rendement de ces réactions est de l'ordre de 90%.

En faisant réagir les composés de formule IV avec les composés de formule V, en présence de l'acide paratoluènesulfonique, on obtient les composés de formule VI avec un rendement de 90%.

Pour obtenir les bases de Schiff de formule VIII, les composés de formule VI sont condensés à chaud avec un aldéhyde de formule VII dans un solvant organique aromatique tel que le toluène.

Les composés des formules X_{A} et X_{B} sont obtenus à partir des composés de l'invention grâce à une β-élimination, inconnue jusqu'à présent dans ce domaine. Cette opération est basée sur la réactivité particulière du proton porté par le carbone de la fonction "thioacétal". Cette méthode de clivage qui consiste à traiter les composés de formules I_{A} et I_{B} par 2 équivalents de n-butyllithium ou d'un autre réactif chimique équivalent, à une température entre -20°C à -80°C, permet l'obtention des azétidinones, X_{A} et X_{B}, quantitativement et très facilement.

Les exemples suivants, donnés à titre non-limitatif, illustrent l'invention.

Les points de fusion indiqués sont mesurés au microscope à platine chauffante Reichert. Les spectres de résonance magnétique nucléaire (RMN) du proton et du ¹³C ont été enregistrés sur un spectomètre Bruker (WP 200).

### EXEMPLE 1

### Chlorhydrate de D-glucosamine propanedithioacétal

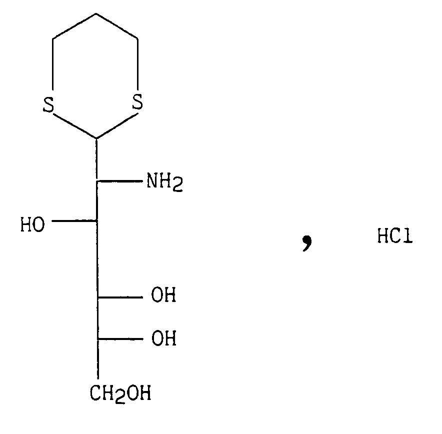

50 g de chlorhydrate de D-glucosamine sont dissous dans 200 ml d'acide chlorhydrique concentré refroidi à 0°C et saturé d'acide chlorhydrique gazeux.

On ajoute 30 ml de propane-1,3-dithiol et 50 ml de dichlorométhane et on laisse 2 heures à température ambiante sous agitation, puis une nuit à 60°C.

On ajoute 500 ml d'eau et ensuite on décante le dichlorométhane qui contient le propane-1,3-dithiol en excès. On lave encore deux fois avec 100 ml de dichlorométhane.

La phase aqueuse est neutralisée par du carbonate de plomb. On essore sur Büchner, et on lave deux fois par 100 ml d'eau. Ensuite, on concentre sous vide. On ajoute 500 ml d'éthanol bouillant et on filtre sur Büchner. Après refroidissement, les cristaux obtenus sont essorés puis rincés à l'éthanol (0°C).
Rendement : 83%
Point de fusion : 115-117°C (éthanol).

### EXEMPLE 2

### 3,4,5,6-di-O-isopropylidène D-glucosamine propanedithioacétal

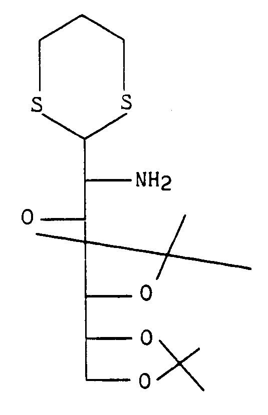

17 g du composé de l'exemple 1 sont dissous dans 45 ml de diméthylformamide et 50 ml de 2,2-diméthoxy propane. On ajoute 5 g d'acide paratoluènesulfonique. On chauffe sous vide de 30 cm de Hg à 70°C, pendant 1 heure 30 minutes dans un rotavapor Büchi ®, puis après refroidissement rapide, on ajoute 200 ml d'hexane, 20 g de bicarbonate de sodium et 10 g de glace et on agite. On extrait 3 fois à l'hexane.

La solution dans l'hexane est séchée et évaporée à sec. Le produit obtenu cristallise facilement dans l'hexane froid.
Rendement : 91%
Point de fusion : 79-80°C (hexane)
Pouvoir rotatoire : (C=1,3% dans le CHCl₃):

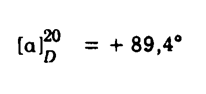

### EXEMPLE 3

### Préparation de 1-(2'-désoxy 2'-dithianyl 3',4';5',6'-di-O-isopropylidène D-glucose) 3β-méthoxy 4β-styryl azétidin-2-one (1)

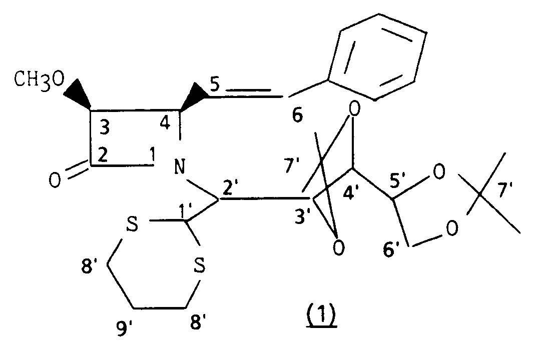

### et de 1-(2'-désoxy 2'-dithianyl 3',4';5',6'-di-O-isopropylidène D-glucose) 3α-méthoxy 4α-styryl azétidin-2-one (2)

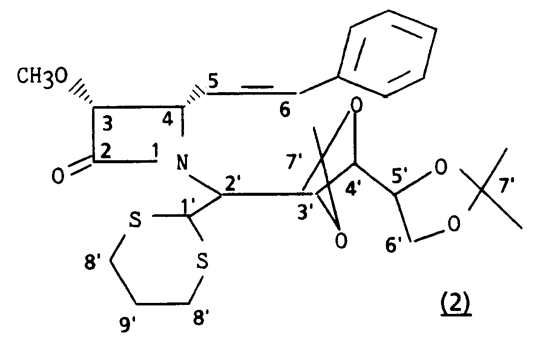

7 g du composé de l'exemple 2 et 2,52 ml d'aldéhyde cinnamique sont dissous dans 6 ml de toluène anhydre. Le mélange est chauffé à 60°C pendant 20 minutes puis, l'eau formée est éliminée par entraînement avec le toluène sous basse pression. L'imine ainsi formée est diluée dans 15 ml de toluène anhydre et on ajoute 6,7 ml de triéthylamine et 2,2 ml du chlorure d'acide méthoxyacétique. La solution est agitée 45 minutes à température ambiante. Le mélange réactionnel est alors versé sur une solution aqueuse saturée de chlorure d'ammonium à 0°C. La phase organique est reprise avec du dichlorométhane et lavée successivement avec une solution aqueuse saturée en chlorure de sodium, séchée sur sulfate de magnésium, filtrée et évaporée sous pression réduite. Une cristallisation dans un mélange hexane-dichlorométhane permet d'isoler 4,8 g de l'isomère 3β4β. Les eaux mères sont chromatographiées sur plaques préparatives de silice en utilisant comme éluant un mélange d'acétate d'éthyle et d'hexane (4:6 V/V) pour séparer les isomères 3α,4α et 3β,4β.
- Rendement :: Isomère 3α,4α : 34%
Isomère 3β,4β : 57%

### Constantes physico-chimiques :

### A. 1-(2'-désoxy 2'-dithianyl 3',4';5',6' di-O-isopropylidène D-glucose) 3β-méthoxy-4β-styryl azétidin-2-one (1)

- Point de fusion : 206°C (hexane-dichlorométhane)
- Pouvoir rotatoire : (C=1% CHCl₃) :

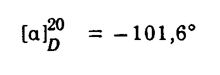

| - Analyse élémentaire : | | | | |
|---|---|---|---|---|
| | C% | H% | N% | S% |
| Théorie : | 60,53 | 6,96 | 2,61 | 11,97 |
| Trouvé : | 60,41 | 6,80 | 2,63 | 12,06 |

Spectre de résonance magnétique nucléaire du proton (solvant CDCl₃) δ ppm: 1,20-1,28-1,36-1,53 (12H, CH₃ᵢₛₒₚᵣ, 4s); 1,9-2,2 (2H,H_{9'}, m); 2,7 (2H,H_{8'},m); 2,9-3,2 (2H,H_{8'},m); 3,46 (3H,OCH₃,s); 3,8-4,4 (7H, H_{1'-6'},m); 4,57 (1H,H₄,dd,J₄₋₃=5Hz, J₄₋₅=9Hz); 4,76 (1H,H₃,d,J₃₋₄=5Hz); 6,45 (1H,dd,H₅,J₅₋₄=9Hz, J₅₋₆=16Hz); 7,3-7,6 (5H,CH_{Ph},m).
Spectre de résonance magnétique nucléaire du ¹³C (solvant CDCl₃) δ ppm : 25,2 (CH₃ᵢₛₒₚᵣ); 25,6 (C_{9'}); 26,4 (CH₃ᵢₛₒₚᵣ); 26,6 (CH₃ᵢₛₒₚᵣ); 27 (CH₃ᵢₛₒₚᵣ); 27,2 (C_{8'}); 44,4 (C_{2'}); 54,1 (C_{1'}); 58,7 (OCH₃); 62,3 (C₄); 67,7 (C_{6'}); 76,9 77,3 79 (C_{3'},C_{4'},C_{5'}); 85,4 (C₃); 109 110 (C_{7'}); 126 126,9 128,1 128,6 134,9 (CH_{Ph},C_{5,}C₆); 136,4 (C_{Ph}); 168,9 (CO).

### B. 1-(2'-désoxy 2'-dithianyl 3',4';5',6'-di-O-isopropylidène D-glucose) 3α-méthoxy 4α-styryl azétidin-2-one (2)

- Point de fusion : 155°C (hexane dichlorométhane)
- Pouvoir rotatoire : (C=1% CHCl₃) :

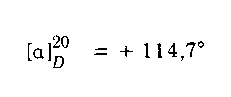

| - Analyse élémentaire : | | | | |
|---|---|---|---|---|
| | C% | H% | N% | S% |
| Théorie : | 60,53 | 6,96 | 2,61 | 11,97 |
| Trouvé : | 60,51 | 7,25 | 2,53 | 12,04 |

Spectre de résonance magnétique nucléaire du proton (solvant CDCl₃) δ ppm: 1,35-1,36-1,49-1,58 (12H,CH₃ᵢₛₒₚᵣ,4s), 1,9-2,2 (2H,m,H_{9'},m); 2,5-2,7 (2H,H_{8'},m); 3,09 (2H,H_{8'},m); 3,46 (3H,OCH₃,s); 3,62 (1H,H_{4'}, dd,J_{4'-3'}=8Hz, J_{4'-5'}=8Hz); 3,90-4,22 (4H,H_{1'}, H_{5'},H_{6'},m); 4,60 (1H, H_{2'},dd,J_{2'-3'}=1,5Hz, J_{2'-1'}=11Hz); 4,69-4,78 (2H,H₃,dd,J₃₋₄=5Hz,H₄,dd, J₄₋₃=5Hz, J₄₋ ₅=10Hz); 4,91 (1H,H_{3'}, dd,J_{3'-2'}=1,5Hz, J_{3'-4'}=8Hz); 6,61 (1H, H_{5'},dd,J₅₋ ₄=10Hz, J₅₋₆=15,5Hz); 6,72 (1H,H₆,d,J₆₋₅=15,5Hz); 7,3-7,5 (5H, CH_{Ph},m).
Spectre de résonance magnétique nucléaire du ¹³C (solvant CDCl₃) δ ppm : 25 (CH₃ᵢₛₒₚᵣ); 25,3 25,6 (C_{9'},C_{8'}); 25,9 (CH₃ᵢₛₒₚᵣ); 26,1 (C_{8'}); 26,3 (CH₃ᵢₛₒₚᵣ); 27,5 (CH₃ᵢₛₒₚᵣ); 43,7 (C_{2'}); 51,8 (C_{1'}); 58,6 (OCH₃); 63 (C₄); 67,5 (C_{6'}); 77,1 77,7 78,5 (C_{3'}, C_{4'}, C_{5'}); 85,3 (C₃); 109,4 (C_{7'}); 109,9 (C_{7'}); 123,9 126,6 128,1 128,6 135,8 (CH_{Ph}, C₅, C₆); 136,2 (C_{Ph}); 167,8 (CO).

### EXEMPLE 4

### 3β-méthoxy 4β-styryl azétidin-2-one

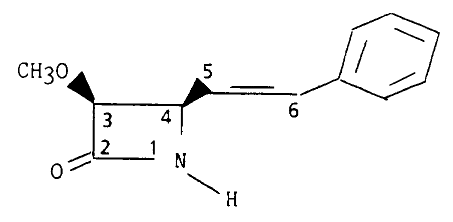

2,6 g de 1-(2'-désoxy 2'-dithianyl 3',4';5',6' di-O-isopropylidène D-glucose) 3β-méthoxy 4β-styryl azétidin-2-one composé de l'exemple 3, sont dissous dans 60 ml de tétrahydrofurane anhydre.

A -40°C, 8 ml de n-butyllithium (1,2M dans l'hexane) sont additionnés lentement. La solution est agitée pendant 20 minutes puis versée sur une solution aqueuse saturée en chlorure d'ammonium à 0°C. Le pH est ramené à 7 par addition d'acide acétique. La phase organique est décantée et lavée avec une solution aqueuse saturée en chlorure de sodium, séchée sur sulfate de magnésium anhydre, filtrée puis évaporée. Le résidu est chromatographié sur colonne de silice en utilisant comme éluant un mélange d'acétate d'éthyle et d'hexane (3:7 V/V) pour obtenir le produit attendu pur.
Rendement : 90%
Point de fusion : 126°C (hexane-dichlorométhane)
Pouvoir rotatoire (C=1% dans CHCl₃) :

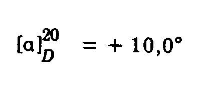

| - Analyse élémentaire : | | | |
|---|---|---|---|
| | C% | H% | N% |
| Théorie : | 70,91 | 6,45 | 6,89 |
| Trouvé : | 70,87 | 6,35 | 6,57 |

Spectre de résonance magnétique nucléaire du proton (solvant CDCl₃) δ ppm: 3,44 (3H,OCH₃,m); 4,38 (1H,H₄,dd,J₄₋₃=4,8Hz, J₄₋₅=8,3Hz); 4,64 (1H,H₃,dd,J₃₋₄=4,8Hz, J₃₋₁=2Hz); 6,26 (1H,H₅,J₅₋₄=8,3Hz, J₅₋₆=16Hz); 6,56 (1H,NH); 6,65 (1H,H₆,J₆₋₅=16Hz); 7,2-7,5 (5H,CH_{Ph},m).
Spectre de résonance magnétique nucléaire du ¹³C (solvant CDCl₃) δ ppm: 56,9 (C₄); 58,5 (CH₃O); 86,8 (C₃); 125,04 (C₆); 126,7 (CH_{Ph}); 128,1 (CH_{Ph}); 128,7 (CH_{Ph}); 134,8 (C₅); 136,36 (C_{Ph}); 168 (CO).

### EXEMPLE 5

### 3a-méthoxy 4a-styryl azétidin-2-one

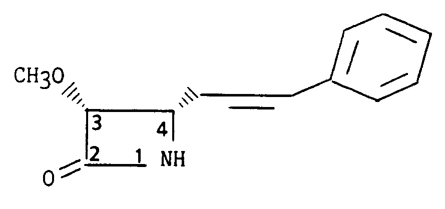

Ce composé a été préparé à partir de la 1-(2'-désoxy 2'-dithianyl 3',4';5',6'-di-O-isopropylidène D-glucose) 3a-méthoxy 4a-styryl azétidin-2-one selon le procédé décrit dans l'exemple 4.
Pouvoir rotatoire (C=1% dans CHCl₃) :

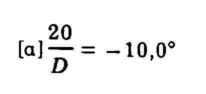

### EXEMPLE 6

### 3,4;5,6-di-O-isopropylidène D-glucosamine diéthyldithioacétal

### STADE A

### Chlorhydrate de D-glucosamine diéthyldithioacétal

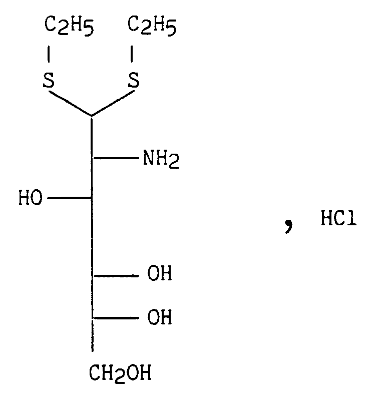

20 g de chlorhydrate de D-glucosamine sont dissous dans 80 ml d'acide chlorhydrique concentré, refroidi à 0°C et saturé d'acide chlorhydrique gazeux. On ajoute 100 mg d'Aliquat 336 ® (Aldrich) et 30 ml d'éthylthiol. On laisse 2 heures à 0°C puis une nuit à température ambiante.

On ajoute du dichlorométhane et on décante la phase organique qui contient l'éthylthiol en excès. On lave ensuite deux fois encore avec le dichlorométhane. On ajoute du chlorure de méthylène et on décante la phase organique qui contient l'éthylthiol en excès. On lave encore deux fois avec le dichlorométhane.

La phase aqueuse est neutralisée par du carbonate de plomb. On essore sur Büchner. On chasse l'eau sous vide. Le résidu est repris à l'éthanol. On filtre sur Büchner. La solution contenant le produit attendu est évaporée à sec.
Point de fusion : 75-76°C (éthanol)

### STADE B

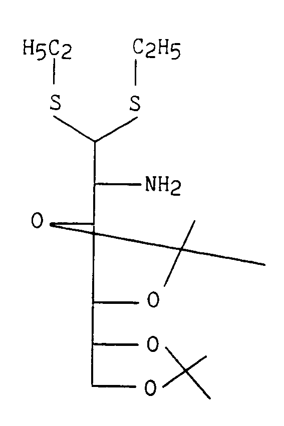

Le résidu obtenu au stade précédent est dissous dans 20 ml de diméthylformamide. Ensuite, le 3,4;5,6-di-O-isopropylidène D-glucosamine diéthyldithioacétal est obtenu selon le procédé décrit dans l'exemple 2. Rendement global : 59%.

### EXEMPLE 7

### Préparation de 1-(2'-désoxy 2'-diéthyldithioacétal 3',4';5',6'-di-O-isopropylidène-D-glucose) 3β-méthoxy 4β-styryl azétidin-2-one (3)

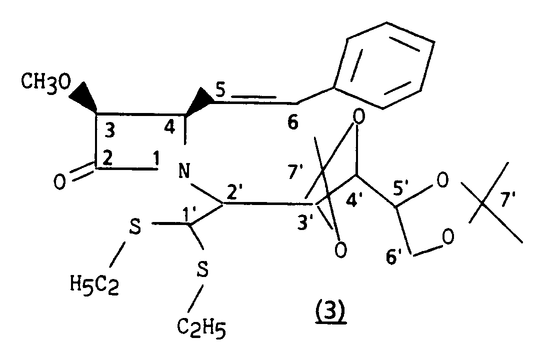

### et de 1-(2'-désoxy 2'-diéthyldithioacétal 3',4';5',6'-di-O-isopropylidène D-glucose) 3α-méthoxy 4α-styryl azétidin-2-one (4)

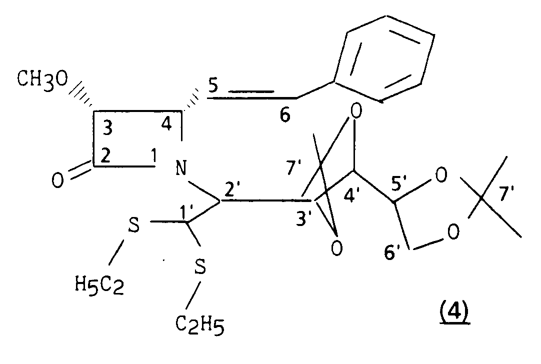

Ces composés ont été préparés à partir du composé obtenu dans l'exemple 6 et selon le procédé décrit dans l'exemple 3.

### Constantes physico-chimiques :

### A. 1-(2'-désoxy 2'-diéthyldithioacétal 3',4';5',6'-di-O-isopropylidène D-glucose) 3β-méthoxy 4β-styryl azétidin-2-one (3)

- Point de fusion : 71-73,5°C (acétone-eau)
- Pouvoir rotatoire : (C=0,98% CHCl₃)

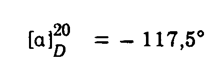

| - Analyse élémentaire : | | | | |
|---|---|---|---|---|
| | C% | H% | N% | S% |
| Théorie : | 60,95 | 7,49 | 2,54 | 11,62 |
| Trouvé : | 60,98 | 7,48 | 2,71 | 11,62 |

Spectre de résonance magnétique nucléaire du proton (solvant CDCl₃) δ ppm: 1,19-3,33 (18H CH_{3isopr.} + 2CH₃CH₂, m) ; 2,6-2,85 (4H 2S-CH₂CH₃, m) ; 3,40 (3H,OMe,s) ; 3,7 (1H,H_{4'},t,J_{4',5'}=J_{4',3'}=7Hz) ; 3,89-4,17 (4H,H_{3'},_{4'},_{5'},_{6'a,} m) ; 4,33 (1H,H_{6'b}, d, J_{6'a,6'b}=9,5Hz) ; 4,62 (1H,H₄,dd,J_{4,3}=5Hz,J_{4,5}=9Hz) ; 4,65-4,68 (2H,H₃,H_{3'},m) ; 6,4 (1H,H₅, dd,J_{5,6}=16Hz,J_{5,4}=9Hz) ; 6,63 (1H,H₆, J_{6,5}=16Hz,d) ; 7,18-7,43 (5H arom,m)

### B. 1-(2'-désoxy 2'-diéthyldithioacétal 3',4';5',6'-di-O-isopropylidène D-glucose) 3α-méthoxy 4α-styryl azétidin-2-one 4

- Point de fusion : 81-84°C (acétone-eau)
- Pouvoir rotatoire : (C=1,15% CHCl₃)

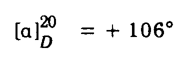

| - Analyse élémentaire : | | | | |
|---|---|---|---|---|
| | C% | H% | N% | S% |
| Théorie : | 60,95 | 7,49 | 2,54 | 11,62 |
| Trouvé : | 60,76 | 7,53 | 2,65 | 11,63 |

Spectre de résonance magnétique nucléaire du proton (solvant CDCl₃) δ ppm: 1,2 (6H,2CH₃CH₂S,t) ; 1,32;1,39;1,42;1,58 (12H,CH₃,_{isopr.}4s) ; 2,57-2,75 (4H,2S-CH₂CH₃,m) ; 3,5 (3H,OCH₃,s) ; 3,6 (1H,H_{4'}, t,J_{4',5'}=J_{4',3'}=7,5Hz) ; 3,93-4,23 (5H,H_{1',2'},_{5',6'a,6'b,m}) ; 4,7 (2H, H₃, H₄,s large) ; 4,95 (1H,H_{3'},dd,J_{3',4'}=7,5 Hz J_{3',2'}=2Hz) ; 6,7-6,73 (2H, H₅, H₆,s large) ; 7,3-7,5 (5H arom,m).

### EXEMPLE 8

### Préparation de 1-(2'-désoxy 2'-dithianyl 3',4';5',6'di-O-isopropylidène-D-glucose) 3β-méthoxy 4β-[2-(fur-2-yl) éthylèn-1-yl] azétidin-2-one (5)

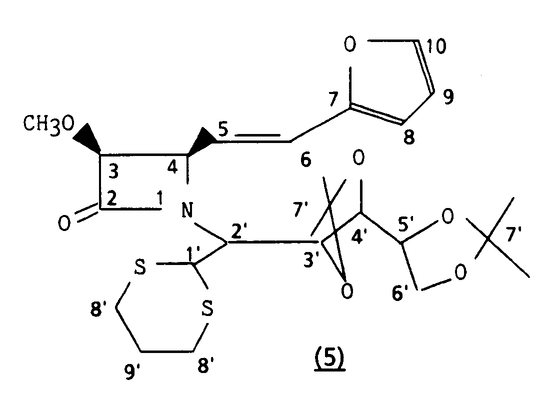

### et de 1-(2'-désoxy 2'-dithianyl 3',4';5',6'-di-O-isopropylidène D-glucose) 3α-méthoxy 4α-[2-(fur-2-yl) éthylèn-1-yl] azétidin-2-one (6)

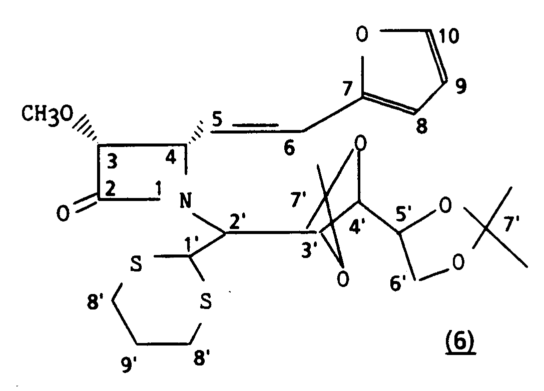

Les deux composés ont été préparés selon le procédé décrit dans l'exemple 3 mais en utilisant la 3-(fur-2-yl) acrylaldéhyde au lieu de l'aldéhyde cinnamique. Les isomères ont été séparés selon la méthode décrite dans l'exemple 3.
- Rendement : Isomère 3α4α :32,12 %
   Isomère 3β4β :48,18 %

### Constantes physico-chimiques

### A. 1-(2'-désoxy 2'-dithianyl 3',4';5',6'di-O-isopropylidène D-glucose) 3β-méthoxy 4β-[2-(fur-2-yl) éthylèn-1-yl] azétidin-2-one (5)

- Point de fusion : 185-188°C (Méthanol)
- Pouvoir rotatoire : (C= 2,28 % CHCl₃) :

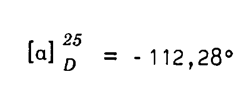

| - Analyse élémentaire : | | | | |
|---|---|---|---|---|
| | C% | H% | N% | S% |
| Théorie : | 57,13 | 6,71 | 2,66 | 12,17 |
| Trouvé : | 57,09 | 6,60 | 2,71 | 12,15 |

Spectre de résonance magnétique nucléaire du proton (solvant CDCl₃) δ ppm: 1,23 ; 1,28 ; 1,36 ; 1,53 (12H, 4CH₃ isopr., 4s) : 1,91-2,11 (2H, H_{8'} m) ; 2,56-2,7 (2H, H_{7'}, m) ; 2,91-3,13 (2H, H_{7'}, m) ; 3,46 (3H, OCH₃, s) ; 3,72-3,82 (1H, H_{4'}, m) ; 4,00-4,23 (4H, H_{1'}, H_{5'}, 2H_{6'}, m) 4,42-4,51 (2H, H_{2'}, H₄, m) ; 4,62-4,74 (2H, H₃,H_{3'}, m) ; 6,27 (1H, H₈, dd, J₈₋₉ = 3H_{z}) ; 6,29 (1H, H₅, dd J₅₋₆ = 16H_{z} ; J₅₋₄ = 9H_{z}) ; 6,37 (1H, H₉, dd, J₉₋₁₀ = 1,5 H_{z}, J₈₋₉ = 3H_{z}) ; 6,46 (1H, H₆, d J₅₋₆ = 16H_{z}) ; 7,36 (1H, H₁₀, d, J₉₋₁₀ = 1,5H_{z}).

### B. 1-(2'-désoxy 2'-dithianyl 3',4';5',6'-di-O-isopropylidène D-glucose) 3α-méthoxy 4α-[2-(fur-2-yl) éthylèn-1-yl] azétidin-2-one (6)

- Pouvoir rotatoire : (C= 1,16 % CHCl₃) :

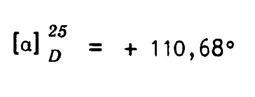

| - Analyse élémentaire : | | | | |
|---|---|---|---|---|
| | C% | H% | N% | S% |
| Théorie : | 57,13 | 6,71 | 2,66 | 12,17 |
| Trouvé : | 57,09 | 6,58 | 2,72 | 12,14 |

Spectre de résonance magnétique nucléaire du proton (solvant CDCl₃) δ ppm: 1,31;1,36;1,48;1,5 (12H, 4CH₃ isopr., s) ; 1,93-2,10 (2H, H_{8'}, m ) ; 2,43-2,68 (2H, H_{7'}, m) ; 2,90-3,23 (2H, H_{7'}, m) ; 3,48 (3H, OCH₃, s) ; 3,63 (1H, H_{4'}, m) ; 3,86-4,20 (4H, H_{1'}, H_{5'}, 2H_{6'}, m) ; 4,55 (1H, H_{2'}, dd) ; 4,60-4,70 (2H, H₃, H₄, m) ; 4,8 (1H, H_{3'}, dd) ; 6,29-6,35 (4H, H₅H₆H₈H₉, m) ; 7,38 (1H, H₁₀, d, J9-10 = 15Hz).

### EXEMPLE 9

### Préparation de 1-(2'-désoxy 2'-diéthyldithioacétal 3',4';5',6'-di-O-isopropylidène-D-glucose) 3β-méthoxy 4β-[2-(fur-2-yl) éthylèn-1-yl] azétidin-2-one (7)

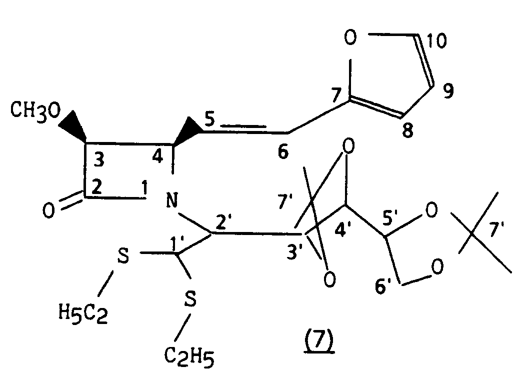

### et de 1-(2'-désoxy 2'-diéthyldithioacétal 3',4';5',6'-di-O-isopropylidène D-glucose) 3α-méthoxy 4α-[2-(fur-2-yl) éthylèn-1-yl] azétidin-2-one (8)

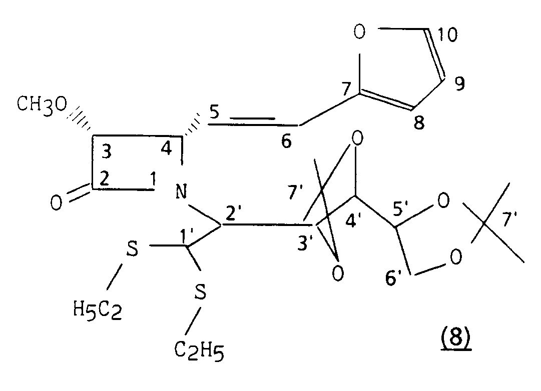

Ces deux composés ont été préparés et séparés selon le procédé décrit dans l'exemple 3 mais en utilisant la 3-(fur-2-yl) acrylaldéhyde et le 3,4;5,6-O-isopropylène D-glucosamine diéthyldithioacétal.
- Rendement : Isomère 3α4α :33,44 %
   Isomère 3β4β :45,60 %

### Constantes physico-chimiques

### A. 1-(2'-désoxy 2'-diéthyldithioacétal 3',4';5',6'-di-O-isopropylidène-D-glucose) 3β-méthoxy 4β-[2-(fur-2-yl) éthylèn-1-yl] azétidin-2-one (7)

- Pouvoir rotatoire : (C= 1,87 % CHCl₃) :

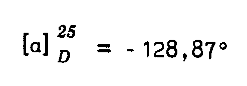

| - Analyse élémentaire : | | | | |
|---|---|---|---|---|
| | C% | H% | N% | S% |
| Théorie : | 57,67 | 7,25 | 2,58 | 11,81 |
| Trouvé : | 57,60 | 7,18 | 2,43 | 11,52 |

Spectre de résonance magnétique nucléaire du proton (solvant CDCl₃) δ ppm: 1,16-1,43 (18H, 6CH₃, m) ; 2,56-2,86, (4H, 2S-CH₂-CH₃, m) ; 3,46 (3H, OCH₃, s) ; 3,7 (1H, H_{4'}, m) ; 3,86-4,16, (4H, H_{1'}, H_{2'}, H_{5'}, H_{6'a}, m) ; 4,25 (1H, H_{6'b,} dd) ; 4,31-4,70 (3H, H₃, H_{3'}, H₄, m) ; 6,27 (1H, H₈, d J₈₋₉ = 3Hz) ; 6,31 (1H, H₅ dd,J₅₋₆ = 16Hz, J₃₋₄ : 8Hz) ; 6,36 (1H, H₉, dd; J₉₋₁₀ = 1,5Hz , J₈₋₉ = 3Hz) ; 6,44 (1H, H₆, d, J₅₋₆ = 16Hz) ; 7,34 (1H, H₁₀, d,J₉₋₁₀ = 1,5Hz).

### B. 1-(2'-désoxy 2'-diéthyldithioacétal 3',4';5',6'-di-O-isopropylidène D-glucose) 3α-méthoxy 4α-[2-(fur-2-yl) éthylèn-1-yl] azétidin-2-one (8)

- Pouvoir rotatoire : (C= 2,13 % CHCl₃) :

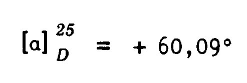

| - Analyse élémentaire : | | | | |
|---|---|---|---|---|
| | C% | H% | N% | S% |
| Théorie : | 57,67 | 7,25 | 2,58 | 11,81 |
| Trouvé : | 57,49 | 7,18 | 2,45 | 11,76 |

Spectre de résonance magnétique nucléaire du proton (solvant CDCl₃) δ ppm: 1,18-1,51, (18H, 6CH₃, m) ; 2,53-2,71 (4H, 2S-CH₂-CH₃, m) ; 3,46 (3H, OCH₃, s) 3,58 (1H, H_{4'}, m) ; 3,80-4,20 (5H, H_{1'}, H_{2'}, H_{5'}, 2H_{6'}, m) ; 4,60-4,82 (2H, H₃, H₄, m) ; 4,90-5,03 (1H, H_{3'}, dd) ; 6,29-6,38 (5H, H₅, H₆, H₈, H₉, m) ; 7,38 (1H, H₁₀,d , J₉₋₁₀ = 1,5).

### EXEMPLE 10

### Préparation de 1-(2'-désoxy 2'-diéthyldithioacétal 3',4';5',6'-di-O-isopropylidène-D-glucose) 3β-méthoxy 4β-(β-méthylstyryl) azétidin-2-one (9)

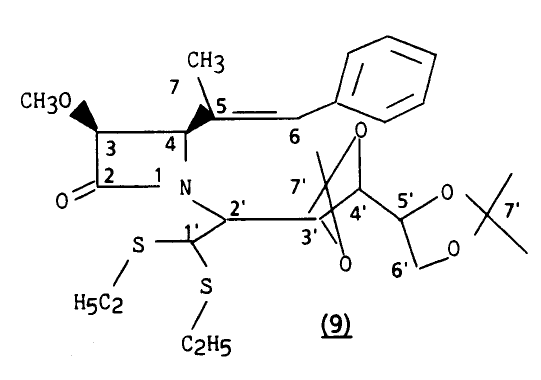

### et de 1-(2'-désoxy 2'-diéthyldithioacétal 3',4';5',6'-di-O-isopropylidène D-glucose) 3α-méthoxy 4α-(β-méthylstyryl) azétidin-2-one (10)

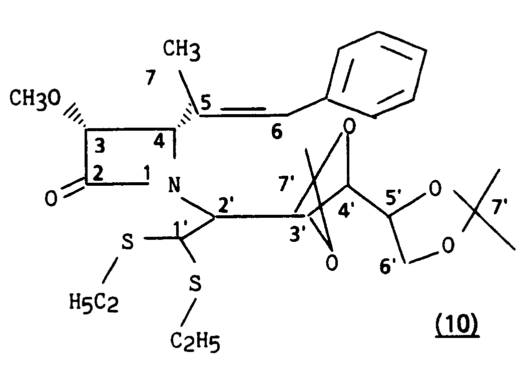

Les deux composés ont été préparés à partir du 3',4';5',6'-di-O-isopropylidène D-glucosamine diéthyldithioacétal et de l'aldéhyde α-méthyl cinnamique, puis séparés selon le procédé décrit dans l'exemple 3.
- Rendement : Isomère 3α4α :13,40 %
   Isomère 3β4β :53,60 %

### Constantes physico-chimiques

### A. 1-(2'-désoxy 2'-diéthyldithioacétal 3',4';5',6'-di-O-isopropylidène-D-glucose) 3β-méthoxy 4β-(β-méthylstyryl) azétidin-2-one (9)

- Pouvoir rotatoire : (C= 1,25 % CHCl₃) :

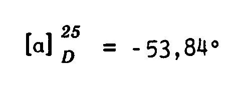

| Analyse élémentaire : | | | | |
|---|---|---|---|---|
| | C% | H% | N% | S% |
| Théorie : | 61,57 | 7,66 | 2,47 | 11,31 |
| Trouvé : | 61,50 | 7,54 | 2,62 | 11,40 |

Spectre de résonance magnétique nucléaire du proton (solvant CDCl₃) δ ppm: 1,1-1,4 (18H, 6CH₃ m) ; 2,05 (3H, CH₃, H₇, s) ; 2,62-2,82 (4H, 2 S-CH₂-CH₃, m) ; 3,45 (3H, OCH₃, s) ; 3,75 (1H, H_{4'}, m) ; 3,95 (1H, H_{5'}, m) ; 4,09-4,35 (4H, H_{1'}, H_{2'}, H₆ₐ, H_{6b}, m) ; 4,61(1H, H₄, d, J₄₋₃ = 5Hz) ; 4,75 (1H, H_{3'}, m) 4,82 (1H, H₃, d J₃₋₄ : 5Hz) ; 6,6 (1H, H₆, s) ; 7,20-7,39 (5H, 1H, m).

### B. 1-(2'-désoxy 2'-diéthyldithioacétal 3',4';5',6'-di-O-isopropylidène D-glucose) 3α-méthoxy 4α-(β-méthylstyryl) azétidin-2-one (10)

- Pouvoir rotatoire : (C= 0,3 % CHCl₃) :

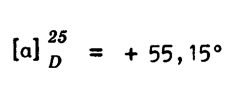

| Analyse élémentaire : | | | | |
|---|---|---|---|---|
| | C% | H% | N% | S% |
| Théorie : | 61,57 | 7,66 | 2,47 | 11,31 |
| Trouvé : | 61,79 | 7,61 | 2,35 | 11,28 |

Spectre de résonance magnétique nucléaire du proton (solvant CDCl₃) δ ppm: 1,1-1,5 (18H, 6CH₃ m) ; 2,05 (3H, H₇, CH₃, s) ; 2,63-2,78 (4H, 2S-CH₂-CH₃, m) ; 3,42 (3H, OCH₃, s) ; 3,78 (1H, H_{4'}, m) ; 3,80-4,21 (5H, H_{1'}, H_{2'}, H_{5'}, H₆ₐ, H_{6b}, m) ; 4,68 (2H, H₃, H₄, m) ; 4,81 (1H, H_{3'}, m) ; 6,73 (1H, H₆, s) 7,21-7,39 (5H, 1H, m).

## Revendications (Revendications pour l'(les) Etat(s) contractant(s) suivant(s): AT, BE, CH, LI, DE, DK, FR, GB, IT, LI, LU, NL, SE)

1. Composés de formule I, constituée par l'ensemble des formules I_{A} et I_{B} : 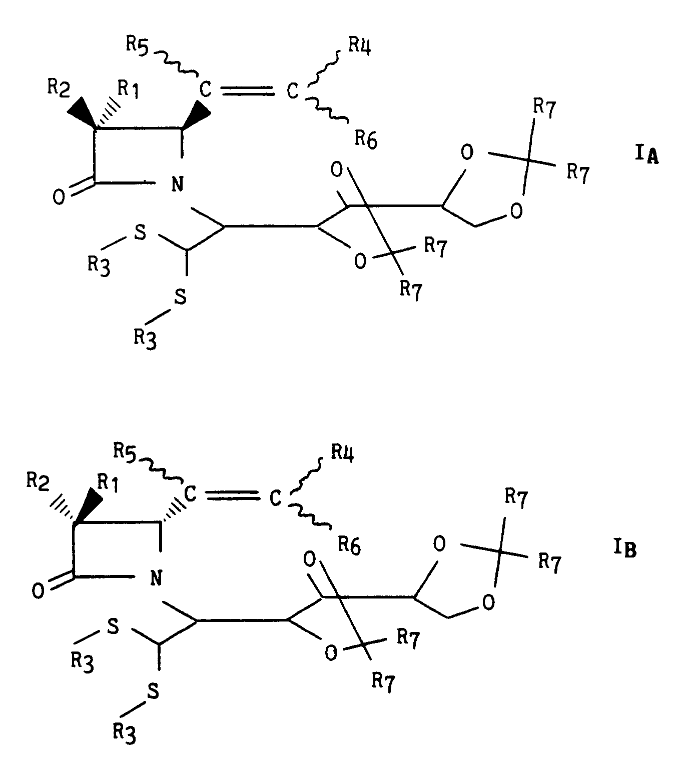 dans lesquelles :
R₁ représente un atome d'hydrogène, un atome d'halogène, un radical alkyle de 1 à 6 atomes de carbone, un radical trifluorométhyle, un radical alcoxy de 1 à 6 atomes de carbone, un radical phényle, phénoxy ou radical benzyle (éventuellement substitués sur le cycle benzénique par un ou plusieurs atomes d'halogène, des radicaux alcoxy de 1 à 6 atomes de carbone ou des radicaux alkyle de 1 à 6 atomes de carbone), un radical benzyloxy, un radical cyano, un radical alcoxycarbonyle de 2 à 7 atomes de carbone, un radical carboxy, ou un radical alkylamino de 1 à 6 atomes de carbone (à condition toutefois que dans ce cas R₂ ne représente jamais simulatément un atome d'hydrogène),
R₂ différent de R₁, représente un atome d'hydrogène, un atome d'halogène, un radical alkyle de 1 à 6 atomes de carbone, un radical trifluorométhyle, un radical alcoxy de 1 à 6 atomes de carbone, un radical phényle, phénoxy ou benzyle (éventuellement substitués sur le cycle benzénique par un ou plusieurs atomes d'halogène, des radicaux alcoxy de 1 à 6 atomes de carbone ou des radicaux alkyle de 1 à 6 atomes de carbone), un radical benzyloxy, un radical cyano, un radical alcoxycarbonyle de 2 à 7 atomes de carbone, un radical carboxy, ou un radical alkylamino de 1 à 6 atomes de carbone, (à condition toutefois que dans ce cas R₁ ne représente jamais simultanément un atome d'hydrogène),
chaque R₃ représente un radical alkyle de 1 à 6 atomes de carbone ou les deux R₃ forment ensemble et avec les atomes de la fonction dithioacétal auxquels ils sont attachés un cycle de 5 à 7 chaînons,
R₄ représente un radical phényle (éventuellement substitué par un ou plusieurs atomes d'halogène, par un ou plusieurs radicaux alkyle de 1 à 6 atomes de carbone, par un radical alcoxy de 1 à 6 atomes de carbone), un radical naphtyle, ou un radical furyle,
R₅ et R₆ identiques ou différents représentent chacun un atome d'hydrogène un radical alkyle de 1 à 7 atomes de carbone, ou un radical aryle (éventuellement substitué par un ou plusieurs atomes d'halogène, des radicaux alcoxy de 1 à 6 atomes de carbone ou des radicaux alkyle de 1 à 6 atomes de carbone),
et
chaque R₇ représente un radical méthyle ou les deux R₇ forment ensemble et avec l'atome de carbone auquel ils sont attachés un noyau cyclohexane ou un noyau cyclopentane.

2. Procédé de préparation des composés de formules I_{A} et I_{B} selon la revendication 1,
caractérisé en ce que l'on condense la D-glucosamine composé de formule II : 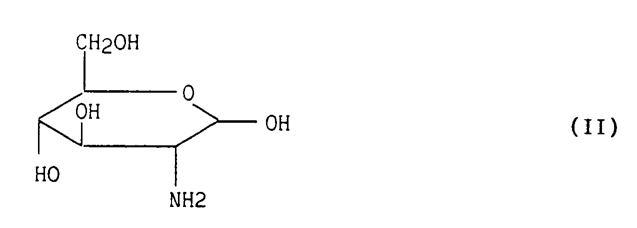
. soit avec une quantité équimolaire d'un composé de formule III_{A} :
**HS(CH₂)**_{**n**}**SH III**_{**A**}
dans laquelle n est égal à 2-4,
. soit avec une quantité au moins double d'un composé de formule III_{B} :
**HSR'₃ III**_{**B**}
dans laquelle R'₃ représente un radical alkyle de 1 à 6 atomes de carbone,
en présence d'acide chlorhydrique concentré pour obtenir un dérivé de D-glucosamine de formule IV: 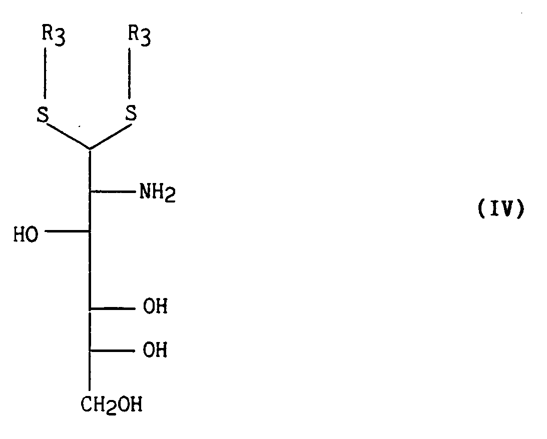 dans laquelle R₃ a la même signification que pour la formule I selon la revendication 1,
sur lequel on fait réagir un composé de formule V : 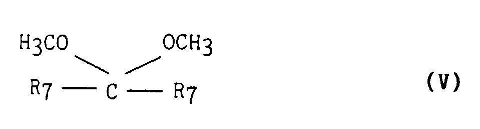 dans laquelle la signification de R₇ reste identique à celle donnée pour la formule I, selon la revendication 1, en présence de l'acide paratoluènesulfonique,
pour obtenir un dérivé de formule VI : 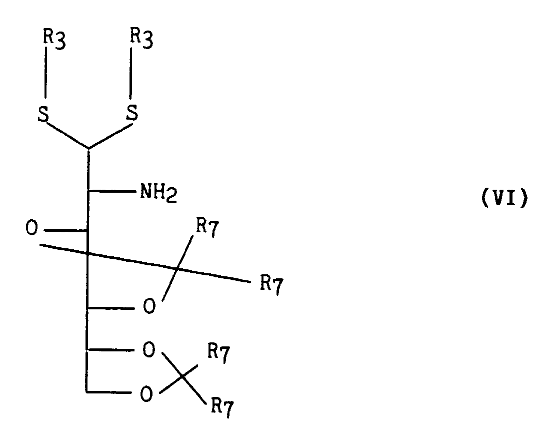 dans laquelle R₃ et R₇ ont la même signification que pour la formule I, selon la revendication 1,
que l'on condense avec un aldéhyde de formule VII : 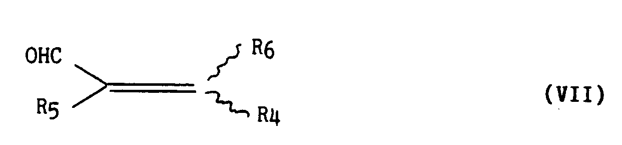 dans laquelle la signification de R₄, R₅, R₆ et R₇ est identique à celle donnée pour la formule I, selon la revendication 1,
pour obtenir une base de Schiff de formule VIII : 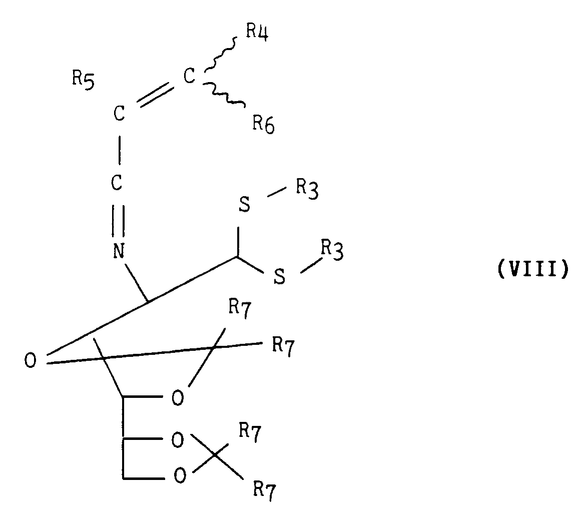 dans laquelle R₃, R₄, R₅, R₆ et R₇ ont la même signification que pour la formule I, selon la revendication 1,
que l'on cyclise ensuite en présence de triéthylamine avec un chlorure d'acide de formule IX: 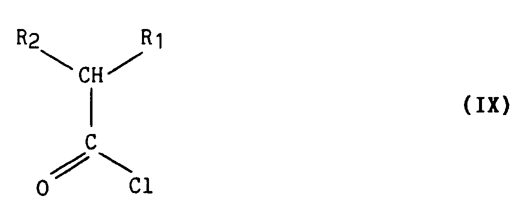 dans laquelle R₁ et R₂ ont la même signification que pour la formule I, selon la revendication 1,
pour obtenir un mélange de monobactames de formule générale I_{A} et I_{B}, que l'on sépare ensuite par cristallisation et/ou par chromatographie préparative.

3. Utilisation des composés I_{A} et I_{B}, selon la revendication 1, comme intermédiaires pour la préparation des monobactames X_{A} et X_{B} : 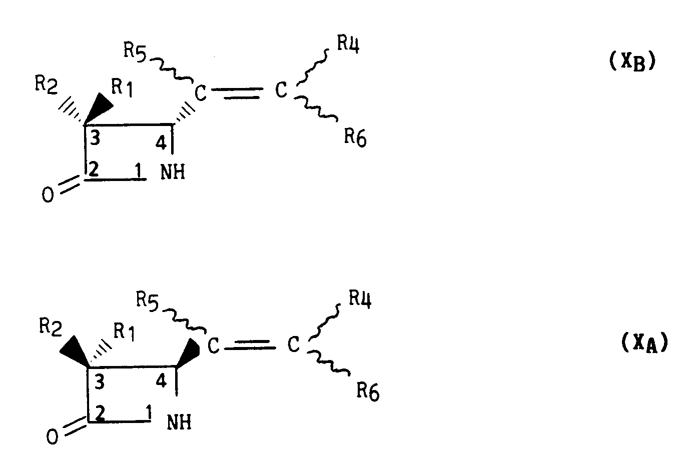 dans lesquelles la signification de R₁, R₂, R₄, R₅ et R₆ reste identique à celle donnée pour la formule I selon la revendication 1.

4. Utilisation des composés de formules I_{A} et I_{B} pour la préparation des composés X_{A} et X_{B}, selon la revendication 3, caractérisée en ce que l'on soumet les composés des formules I_{A} et I_{B} à l'action de n-butyllithium ou d'un autre réactif chimique équivalent, pour obtenir les composés de formules X_{A} et X_{B}.

5. Procédé de préparation des composés X_{A} et X_{B} selon les revendications 3 et 4, caractérisé en ce que l'on soumet les composés de formules I_{A} et I_{B} en solution dans le tétrahydrofurane, ou dans un autre solvant organique équivalent, à une température comprise entre -20°C et -80°C, à l'action du n-butyllithium,
puis qu'on neutralise le milieu réactionnel à l'aide d'un acide minéral, pour obtenir les composés de formules X_{A} et X_{B}.

## Revendications (Revendications pour l'(les) Etat(s) contractant(s) suivant(s): ES)

1. Procédé de préparation des composés de formule I, constituée par l'ensemble des formules I_{A} et I_{B} : 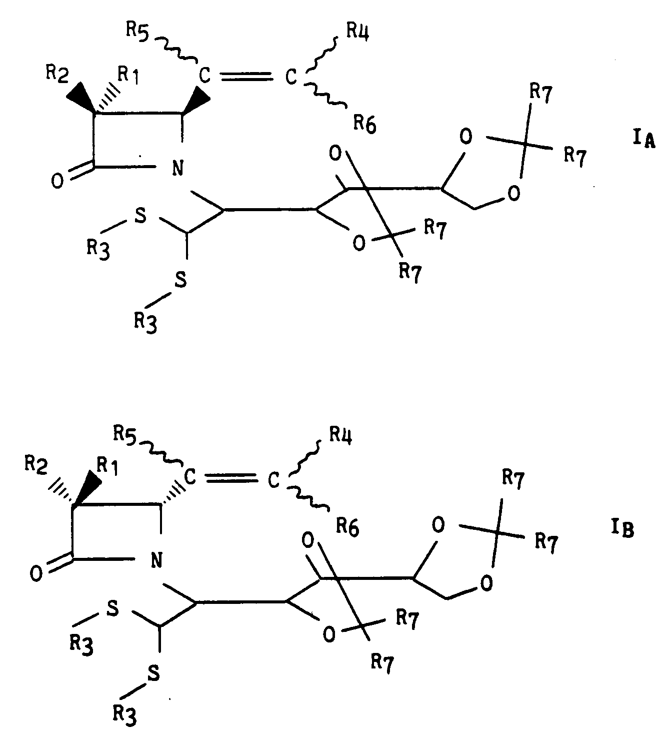 dans lesquelles :
R₁ représente un atome d'hydrogène, un atome d'halogène, un radical alkyle de 1 à 6 atomes de carbone, un radical trifluorométhyle, un radical alcoxy de 1 à 6 atomes de carbone, un radical phényle, phénoxy ou radical benzyle (éventuellement substitués sur le cycle benzénique par un ou plusieurs atomes d'halogène, des radicaux alcoxy de 1 à 6 atomes de carbone ou des radicaux alkyle de 1 à 6 atomes de carbone), un radical benzyloxy, un radical cyano, un radical alcoxycarbonyle de 2 à 7 atomes de carbone, un radical carboxy, ou un radical alkylamino de 1 à 6 atomes de carbone (à condition toutefois que dans ce cas R₂ ne représente jamais simulatément un atome d'hydrogène),
R₂ différent de R₁, représente un atome d'hydrogène, un atome d'halogène, un radical alkyle de 1 à 6 atomes de carbone, un radical trifluorométhyle, un radical alcoxy de 1 à 6 atomes de carbone, un radical phényle, phénoxy ou benzyle (éventuellement substitués sur le cycle benzénique par un ou plusieurs atomes d'halogène, des radicaux alcoxy de 1 à 6 atomes de carbone ou des radicaux alkyle de 1 à 6 atomes de carbone), un radical benzyloxy, un radical cyano, un radical alcoxycarbonyle de 2 à 7 atomes de carbone, un radical carboxy, ou un radical alkylamino de 1 à 6 atomes de carbone, (à condition toutefois que dans ce cas R₁ ne représente jamais simultanément un atome d'hydrogène),
chaque R₃ représente un radical alkyle de 1 à 6 atomes de carbone ou les deux R₃ forment ensemble et avec les atomes de la fonction dithioacétal auxquels ils sont attachés un cycle de 5 à 7 chaînons,
R₄ représente un radical phényle (éventuellement substitué par un ou plusieurs atomes d'halogène, par un ou plusieurs radicaux alkyle de 1 à 6 atomes de carbone, par un radical alcoxy de 1 à 6 atomes de carbone), un radical naphtyle, ou un radical furyle,
R₅ et R₆ identiques ou différents représentent chacun un atome d'hydrogène un radical alkyle de 1 à 7 atomes de carbone, ou un radical aryle (éventuellement substitué par un ou plusieurs atomes d'halogène, des radicaux alcoxy de 1 à 6 atomes de carbone ou des radicaux alkyle de 1 à 6 atomes de carbone),
et
chaque R₇ représente un radical méthyle ou les deux R₇ forment ensemble et avec l'atome de carbone auquel ils sont attachés un noyau cyclohexane ou un noyau cyclopentane,
caractérisé en ce que l'on condense la D-glucosamine composé de formule II : 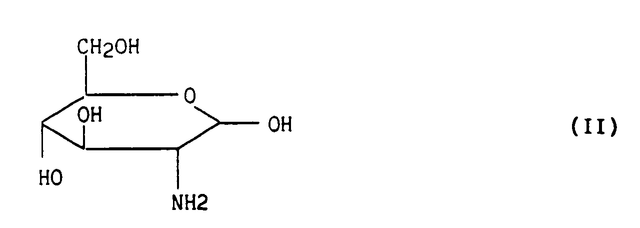
. soit avec une quantité équimolaire d'un composé de formule III_{A} :
**HS(CH₂)**_{**n**}**SH III**_{**A**}
dans laquelle n est égal à 2-4,
. soit avec une quantité au moins double d'un composé de formule III_{B} :
**HSR'₃ III**_{**B**}
dans laquelle R'₃ représente un radical alkyle de 1 à 6 atomes de carbone,
en présence d'acide chlorhydrique concentré pour obtenir un dérivé de D-glucosamine de formule IV: 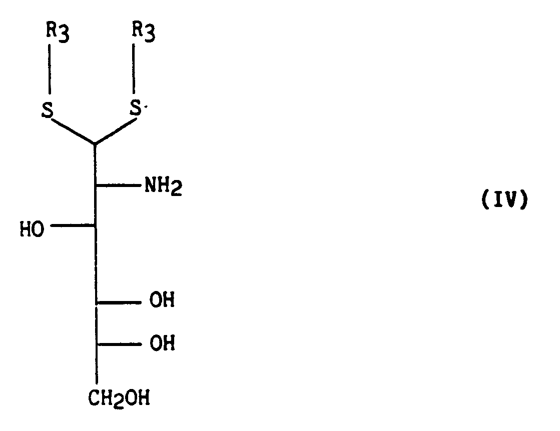 dans laquelle R₃ a la même signification que pour la formule I selon la revendication 1,
sur lequel on fait réagir un composé de formule V : 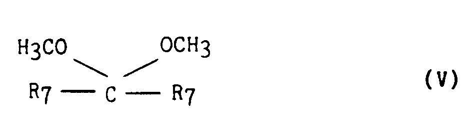 dans laquelle la signification de R₇ reste identique à celle donnée pour la formule I, selon la revendication 1, en présence de l'acide paratoluènesulfonique,
pour obtenir un dérivé de formule VI : 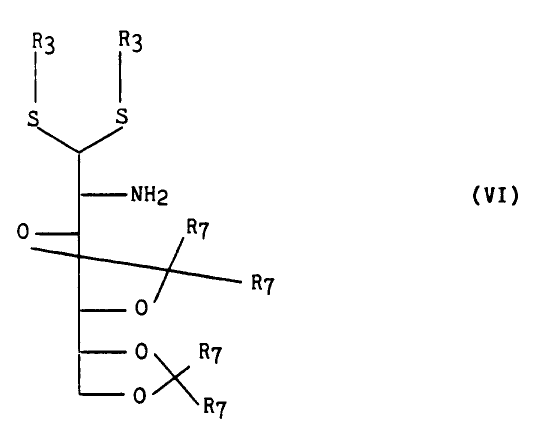 dans laquelle R₃ et R₇ ont la même signification que pour la formule I, selon la revendication 1,
que l'on condense avec un aldéhyde de formule VII : 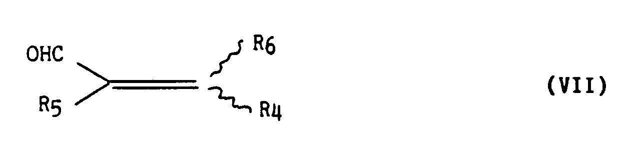 dans laquelle la signification de R₄, R₅, R₆ et R₇ est identique à celle donnée pour la formule I, selon la revendication 1,
pour obtenir une base de Schiff de formule VIII : 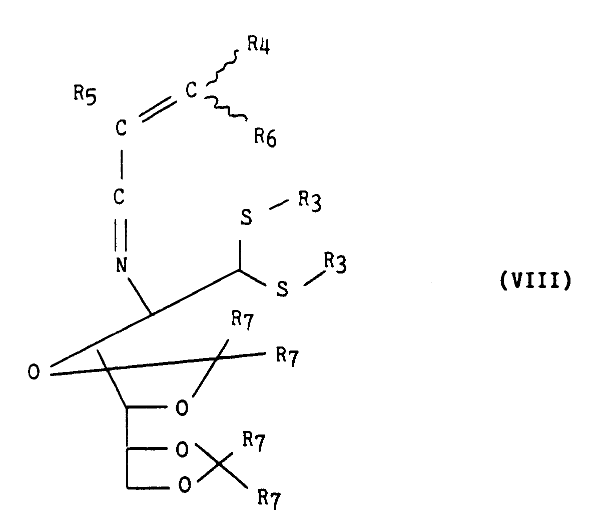 dans laquelle R₃, R₄, R₅, R₆ et R₇ ont la même signification que pour la formule I, selon la revendication 1,
que l'on cyclise ensuite en présence de triéthylamine avec un chlorure d'acide de formule IX: 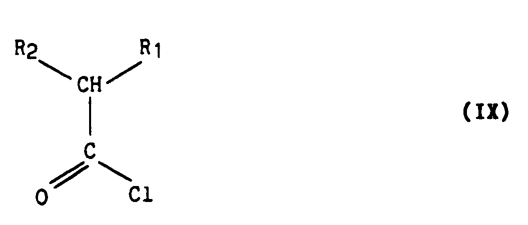 dans laquelle R₁ et R₂ ont la même signification que pour la formule I, selon la revendication 1,
pour obtenir un mélange de monobactames de formule générale I_{A} et I_{B}, que l'on sépare ensuite par cristallisation et/ou par chromatographie préparative.

2. Utilisation des composés I_{A} et I_{B}, selon la revendication 1, comme intermédiaires pour la préparation des monobactames X_{A} et X_{B} : 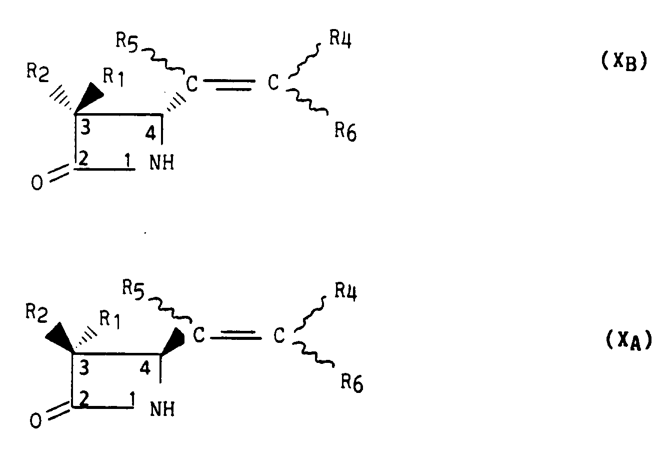 dans lesquelles la signification de R₁, R₂, R₄, R₅ et R₆ reste identique à celle donnée pour la formule I selon la revendication 1.

3. Utilisation des composés de formules I_{A} et I_{B} pour la préparation des composés X_{A} et X_{B}, selon la revendication 2, caractérisée en ce que l'on soumet les composés des formules I_{A} et I_{B} à l'action de n-butyllithium ou d'un autre réactif chimique équivalent, pour obtenir les composés de formules X_{A} et X_{B}.

4. Un procédé de préparation des composés X_{A} et X_{B} selon les revendications 2 et 3, caractérisé en ce que l'on soumet les composés de formules I_{A} et I_{B} en solution dans le tétrahydrofurane, ou dans un autre solvant organique équivalent, à une température comprise entre -20°C et -80°C, à l'action du n-butyllithium,
puis qu'on neutralise le milieu réactionnel à l'aide d'un acide minéral, pour obtenir les composés de formules X_{A} et X_{B}.

## Revendications (Revendications pour l'(les) Etat(s) contractant(s) suivant(s): GR)

1. Composés de formule I, constituée par l'ensemble des formules I_{A} et I_{B} : 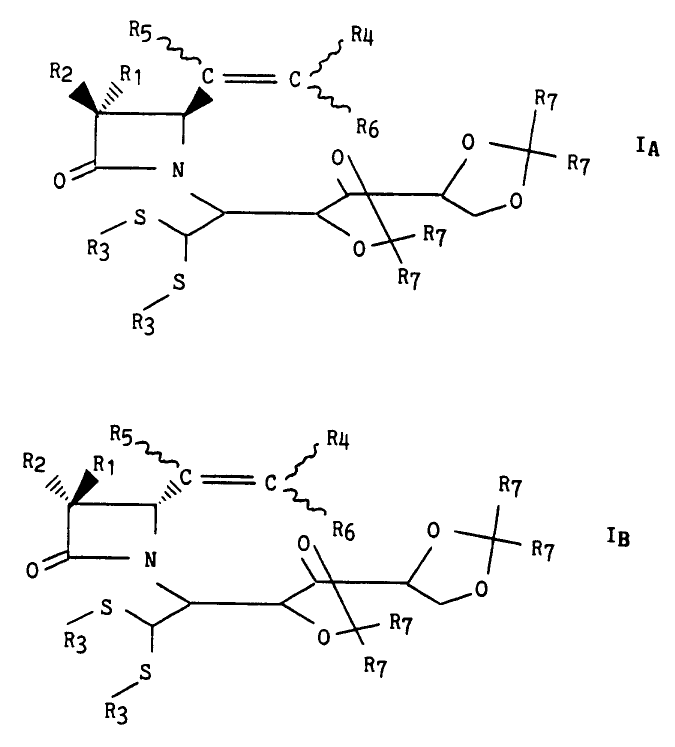 dans lesquelles :
R₁ représente un atome d'hydrogène, un atome d'halogène, un radical alkyle de 1 à 6 atomes de carbone, un radical trifluorométhyle, un radical alcoxy de 1 à 6 atomes de carbone, un radical phényle, phénoxy ou radical benzyle (éventuellement substitués sur le cycle benzénique par un ou plusieurs atomes d'halogène, des radicaux alcoxy de 1 à 6 atomes de carbone ou des radicaux alkyle de 1 à 6 atomes de carbone), un radical benzyloxy, un radical cyano, un radical alcoxycarbonyle de 2 à 7 atomes de carbone, un radical carboxy, ou un radical alkylamino de 1 à 6 atomes de carbone (à condition toutefois que dans ce cas R₂ ne représente jamais simulatément un atome d'hydrogène),
R₂ différent de R₁, représente un atome d'hydrogène, un atome d'halogène, un radical alkyle de 1 à 6 atomes de carbone, un radical trifluorométhyle, un radical alcoxy de 1 à 6 atomes de carbone, un radical phényle, phénoxy ou benzyle (éventuellement substitués sur le cycle benzénique par un ou plusieurs atomes d'halogène, des radicaux alcoxy de 1 à 6 atomes de carbone ou des radicaux alkyle de 1 à 6 atomes de carbone), un radical benzyloxy, un radical cyano, un radical alcoxycarbonyle de 2 à 7 atomes de carbone, un radical carboxy, ou un radical alkylamino de 1 à 6 atomes de carbone, (à condition toutefois que dans ce cas R₁ ne représente jamais simultanément un atome d'hydrogène),
chaque R₃ représente un radical alkyle de 1 à 6 atomes de carbone ou les deux R₃ forment ensemble et avec les atomes de la fonction dithioacétal auxquels ils sont attachés un cycle de 5 à 7 chaînons,
R₄ représente un radical phényle (éventuellement substitué par un ou plusieurs atomes d'halogène, par un ou plusieurs radicaux alkyle de 1 à 6 atomes de carbone, par un radical alcoxy de 1 à 6 atomes de carbone), un radical naphtyle, ou un radical furyle,
R₅ et R₆ identiques ou différents représentent chacun un atome d'hydrogène un radical alkyle de 1 à 7 atomes de carbone, ou un radical aryle (éventuellement substitué par un ou plusieurs atomes d'halogène, des radicaux alcoxy de 1 à 6 atomes de carbone ou des radicaux alkyle de 1 à 6 atomes de carbone),
et
chaque R₇ représente un radical méthyle ou les deux R₇ forment ensemble et avec l'atome de carbone auquel ils sont attachés un noyau cyclohexane ou un noyau cyclopentane.

2. Procédé de préparation des composés de formules I_{A} et I_{B} selon la revendication 1,
caractérisé en ce que l'on condense la D-glucosamine composé de formule II : 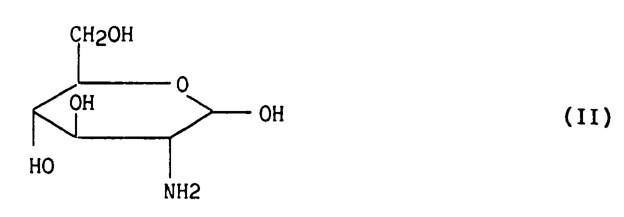
. soit avec une quantité équimolaire d'un composé de formule III_{A} :
**HS(CH₂)**_{**n**}**SH III**_{**A**}
dans laquelle n est égal à 2-4,
. soit avec une quantité au moins double d'un composé de formule III_{B} :
**HSR'₃ III**_{**B**}
dans laquelle R'₃ représente un radical alkyle de 1 à 6 atomes de carbone,
en présence d'acide chlorhydrique concentré pour obtenir un dérivé de D-glucosamine de formule IV: 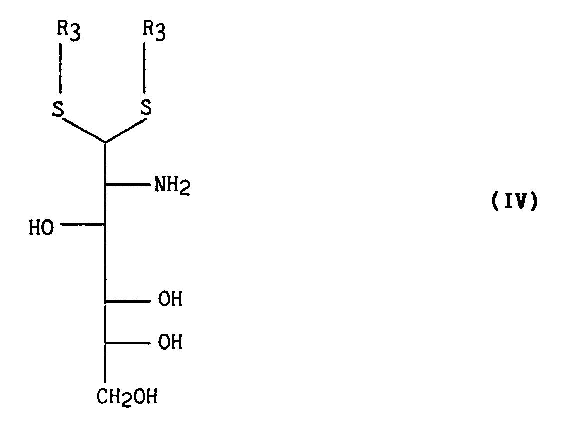 dans laquelle R₃ a la même signification que pour la formule I selon la revendication 1,
sur lequel on fait réagir un composé de formule V : 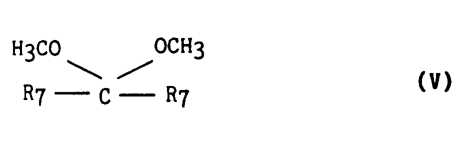 dans laquelle la signification de R₇ reste identique à celle donnée pour la formule I, selon la revendication 1, en présence de l'acide paratoluènesulfonique,
pour obtenir un dérivé de formule VI : 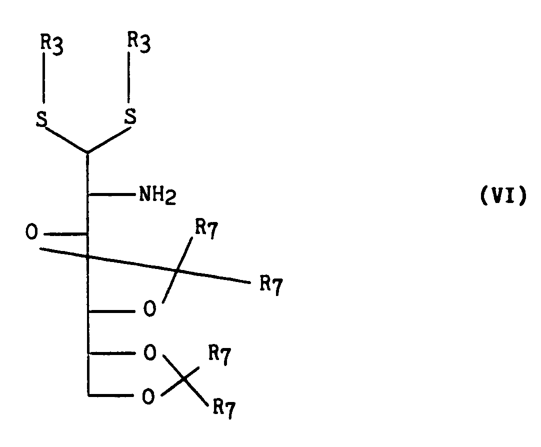 dans laquelle R₃ et R₇ ont la même signification que pour la formule I, selon la revendication 1,
que l'on condense avec un aldéhyde de formule VII : 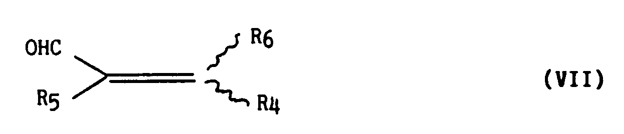 dans laquelle la signification de R₄, R₅, R₆ et R₇ est identique à celle donnée pour la formule I, selon la revendication 1,
pour obtenir une base de Schiff de formule VIII : 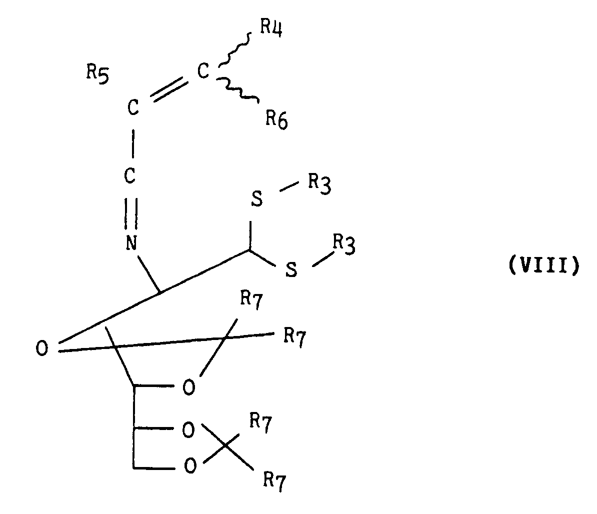 dans laquelle R₃, R₄, R₅, R₆ et R₇ ont la même signification que pour la formule I, selon la revendication 1,
que l'on cyclise ensuite en présence de triéthylamine avec un chlorure d'acide de formule IX: 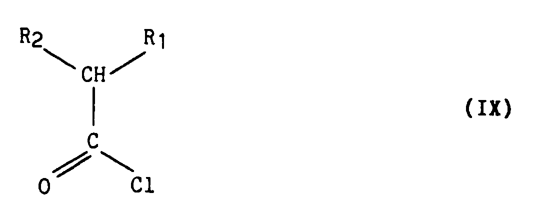 dans laquelle R₁ et R₂ ont la même signification que pour la formule I, selon la revendication 1,
pour obtenir un mélange de monobactames de formule générale I_{A} et I_{B}, que l'on sépare ensuite par cristallisation et/ou par chromatographie préparative.

3. Utilisation des composés I_{A} et I_{B}, selon la revendication 1, comme intermédiaires pour la préparation des monobactames X_{A} et X_{B} : 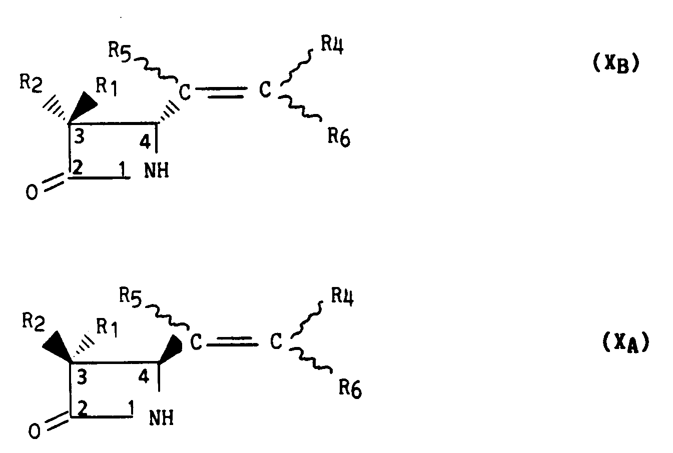 dans lesquelles la signification de R₁, R₂, R₄, R₅ et R₆ reste identique à celle donnée pour la formule I selon la revendication 1.

4. Utilisation des composés de formules I_{A} et I_{B} pour la préparation des composés X_{A} et X_{B}, selon la revendication 3, caractérisée en ce que l'on soumet les composés des formules I_{A} et I_{B} à l'action de n-butyllithium ou d'un autre réactif chimique équivalent, pour obtenir les composés de formules X_{A} et X_{B}.

5. Procédé de préparation des composés X_{A} et X_{B} selon les revendications 3 et 4, caractérisé en ce que l'on soumet les composés de formules I_{A} et I_{B} en solution dans le tétrahydrofurane, ou dans un autre solvant organique équivalent, à une température comprise entre -20°C et -80°C, à l'action du n-butyllithium,
puis qu'on neutralise le milieu réactionnel à l'aide d'un acide minéral, pour obtenir les composés de formules X_{A} et X_{B}.

## Claims (Claims for the following Contracting State(s): AT, BE, CH, DE, DK, FR, GB, IT, LI, LU, NL, SE)

1. Compounds of formula I, constituted by the totality of formulae I_{A} and I_{B}: 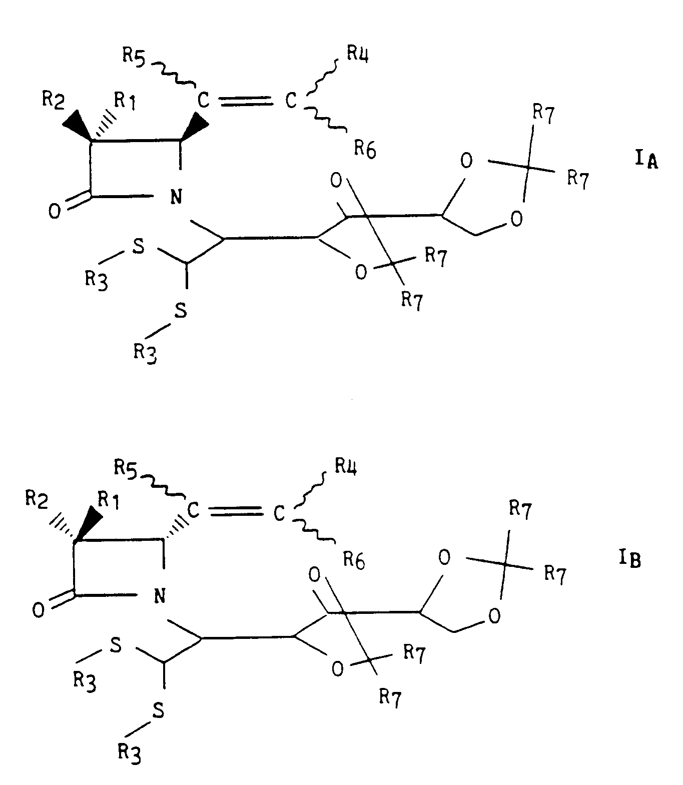 wherein:
R₁ represents a hydrogen atom, a halogen atom, an alkyl radical having from 1 to 6 carbon atoms, a trifluoromethyl radical, an alkoxy radical having from 1 to 6 carbon atoms, a phenyl or phenoxy radical or a benzyl radical (each of which is optionally substituted on the benzene ring by one or more halogen atoms, alkoxy radicals having from 1 to 6 carbon atoms or alkyl radicals having from 1 to 6 carbon atoms), a benzyloxy radical, a cyano radical, an alkoxycarbonyl radical having from 2 to 7 carbon atoms, a carboxy radical, or an alkylamino radical having from 1 to 6 carbon atoms (with the proviso, however, that in that case R₂ never simultaneously represents a hydrogen atom),
R₂, which differs from R₁, represents a hydrogen atom, a halogen atom, an alkyl radical having from 1 to 6 carbon atoms, a trifluoromethyl radical, an alkoxy radical having from 1 to 6 carbon atoms, a phenyl, phenoxy or benzyl radical (each of which is optionally substituted on the benzene ring by one or more halogen atoms, alkoxy radicals having from 1 to 6 carbon atoms or alkyl radicals having from 1 to 6 carbon atoms), a benzyloxy radical, a cyano radical, an alkoxycarbonyl radical having from 2 to 7 carbon atoms, a carboxy radical, or an alkylamino radical having from 1 to 6 carbon atoms (with the proviso, however, that in that case R₁ never simultaneously represents a hydrogen atom),
each R₃ represents an alkyl radical having from 1 to 6 carbon atoms or the two R₃ form together and with the atoms of the dithioacetal function to which they are attached a ring having from 5 to 7 ring members,
R₄ represents a phenyl radical (optionally substituted by one or more halogen atoms, by one or more alkyl radicals having from 1 to 6 carbon atoms, by an alkoxy radical having from 1 to 6 carbon atoms), a naphthyl radical or a furyl radical,
each of R₅ and R₆, which may be identical or different, represents a hydrogen atom, an alkyl radical having from 1 to 7 carbon atoms, or an aryl radical (optionally substituted by one or more halogen atoms, alkoxy radicals having from 1 to 6 carbon atoms or alkyl radicals having from 1 to 6 carbon atoms),
and
each R₇ represents a methyl radical or the two R₇ form together and with the carbon atom to which they are attached a cyclohexane ring or a cyclopentane ring.

2. Process for the preparation of the compounds of formulae I_{A} and I_{B} according to claim 1,
characterised in that D-glucosamine, a compound of formula II: 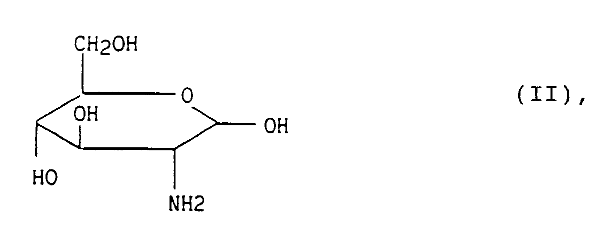 is condensed
. either with an equimolar amount of a compound of formula III_{A}:
HS(CH₂)ₙSH III_{A}
wherein n is 2 to 4,
. or with at least double the amount of a compound of formula III_{B}:
HSR'₃ III_{B}
wherein R'₃ represents an alkyl radical having from 1 to 6 carbon atoms,
in the presence of concentrated hydrochloric acid to obtain a D-glucosamine compound of formula IV: 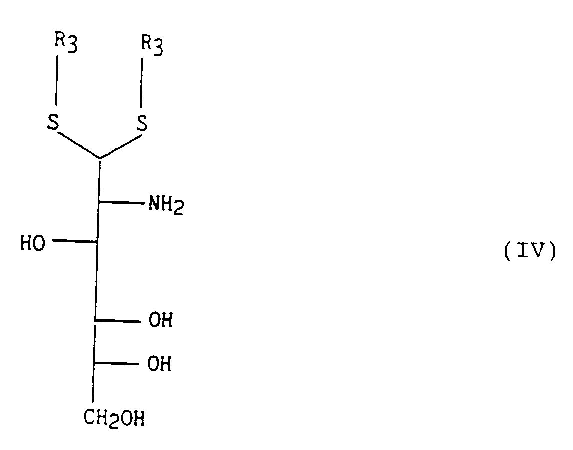 wherein R₃ is as defined for formula I according to claim 1,
with which is reacted a compound of formula V: 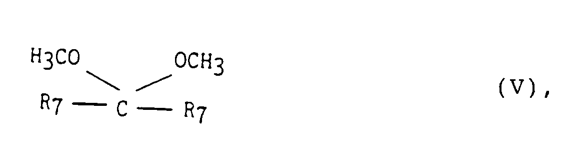 wherein R₇ is as defined for formula I according to claim 1, in the presence of paratoluenesulphonic acid,
to obtain a compound of formula VI: 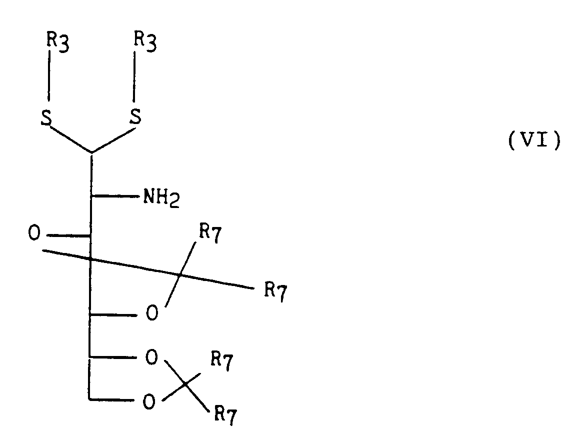 wherein R₃ and R₇ are as defined for formula I according to claim 1,
which is condensed with an aldehyde of formula VII: 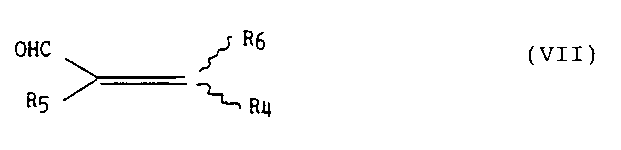 wherein R₄, R₅, R₆ and R₇ are as defined for formula I according to claim 1,
to obtain a Schiff's base of formula VIII: 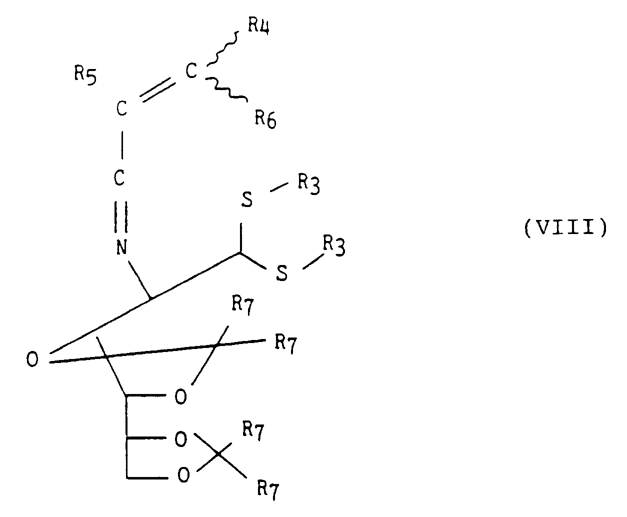 wherein R₃, R₄, R₅, R₆ and R₇ are as defined for formula I according to claim 1,
which is then cyclised in the presence of triethylamine with an acid chloride of formula IX: 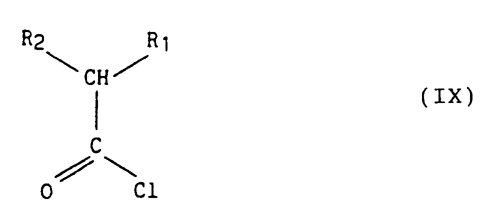 wherein R₁ and R₂ are as defined for formula I according to claim 1,
to obtain a mixture of monobactams of the general formulae I_{A} and I_{B} which is then separated by crystallisation and/or by preparative chromatography.

3. Use of compounds I_{A} and I_{B} according to claim 1 as intermediates for the preparation of the monobactams X_{A} and X_{B}: 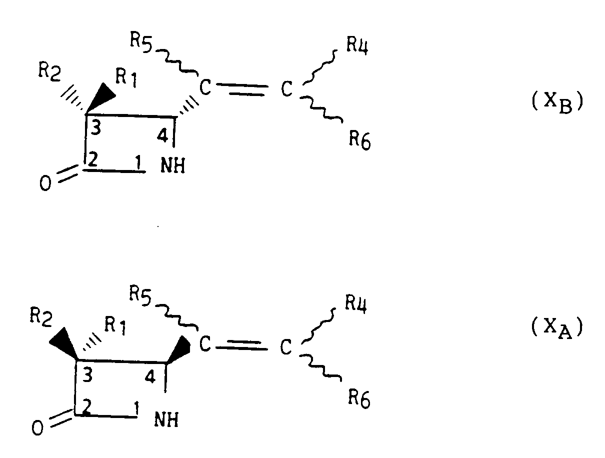 wherein R₁, R₂, R₄, R₅ and R₆ are as defined for formula I according to claim 1.

4. Use of the compounds of formulae I_{A} and I_{B} in the preparation of compounds X_{A} and X_{B} according to claim 3, characterised in that the compounds of formulae I_{A} and I_{B} are subjected to the action of n-butyllithium or another equivalent chemical reagent to obtain the compounds of formulae X_{A} and X_{B}.

5. Process for the preparation of compounds X_{A} and X_{B} according to claims 3 and 4, characterised in that the compounds of formulae I_{A} and I_{B} are subjected, in solution in tetrahydrofuran, or in another equivalent organic solvent, at a temperature of from -20°C to -80°C, to the action of n-butyllithium,
and then the reaction medium is neutralised with a mineral acid to obtain the compounds of formulae X_{A} and X_{B}.

## Claims (Claims for the following Contracting State(s): ES)

1. Process for the preparation of compounds of formula I, constituted by the totality of formulae I_{A} and I_{B}: 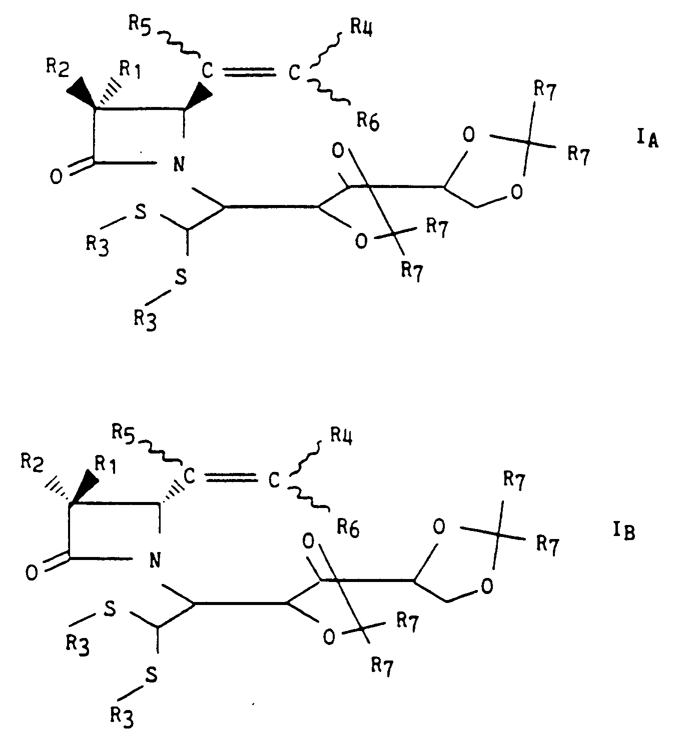 wherein:
R₁ represents a hydrogen atom, a halogen atom, an alkyl radical having from 1 to 6 carbon atoms, a trifluoromethyl radical, an alkoxy radical having from 1 to 6 carbon atoms, a phenyl or phenoxy radical or a benzyl radical (each of which is optionally substituted on the benzene ring by one or more halogen atoms, alkoxy radicals having from 1 to 6 carbon atoms or alkyl radicals having from 1 to 6 carbon atoms), a benzyloxy radical, a cyano radical, an alkoxycarbonyl radical having from 2 to 7 carbon atoms, a carboxy radical, or an alkylamino radical having from 1 to 6 carbon atoms (with the proviso, however, that in that case R₂ never simultaneously represents a hydrogen atom),
R₂, which differs from R₁, represents a hydrogen atom, a halogen atom, an alkyl radical having from 1 to 6 carbon atoms, a trifluoromethyl radical, an alkoxy radical having from 1 to 6 carbon atoms, a phenyl, phenoxy or benzyl radical (each of which is optionally substituted on the benzene ring by one or more halogen atoms, alkoxy radicals having from 1 to 6 carbon atoms or alkyl radicals having from 1 to 6 carbon atoms), a benzyloxy radical, a cyano radical, an alkoxycarbonyl radical having from 2 to 7 carbon atoms, a carboxy radical, or an alkylamino radical having from 1 to 6 carbon atoms (with the proviso, however, that in that case R₁ never simultaneously represents a hydrogen atom),
each R₃ represents an alkyl radical having from 1 to 6 carbon atoms or the two R₃ form together and with the atoms of the dithioacetal function to which they are attached a ring having from 5 to 7 ring members,
R₄ represents a phenyl radical (optionally substituted by one or more halogen atoms, by one or more alkyl radicals having from 1 to 6 carbon atoms, by an alkoxy radical having from 1 to 6 carbon atoms), a naphthyl radical or a furyl radical,
each of R₅ and R₆, which may be identical or different, represents a hydrogen atom, an alkyl radical having from 1 to 7 carbon atoms, or an aryl radical (optionally substituted by one or more halogen atoms, alkoxy radicals having from 1 to 6 carbon atoms or alkyl radicals having from 1 to 6 carbon atoms),
and
each R₇ represents a methyl radical or the two R₇ form together and with the carbon atom to which they are attached a cyclohexane ring or a cyclopentane ring,
characterised in that D-glucosamine, a compound of formula II: 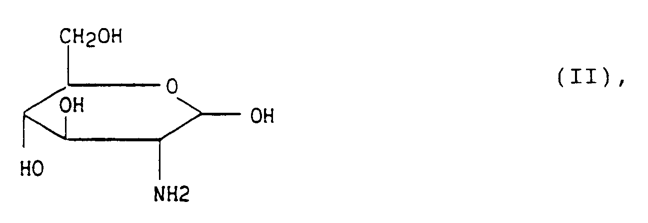 is condensed
. either with an equimolar amount of a compound of formula III_{A}:
HS(CH₂)ₙSH III_{A}
wherein n is 2 to 4,
. or with at least double the amount of a compound of formula III_{B}:
HSR'₃ III_{B}
wherein R'₃ represents an alkyl radical having from 1 to 6 carbon atoms,
in the presence of concentrated hydrochloric acid to obtain a D-glucosamine compound of formula IV: 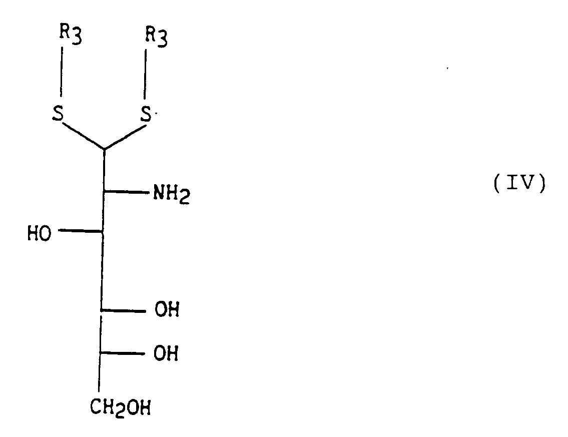 wherein R₃ is as defined for formula I according to claim 1,
with which is reacted a compound of formula V: 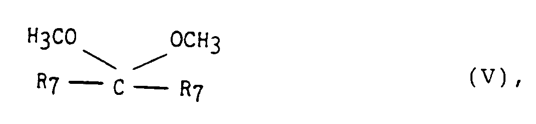 wherein R₇ is as defined for formula I according to claim 1, in the presence of paratoluenesulphonic acid,
to obtain a compound of formula VI: 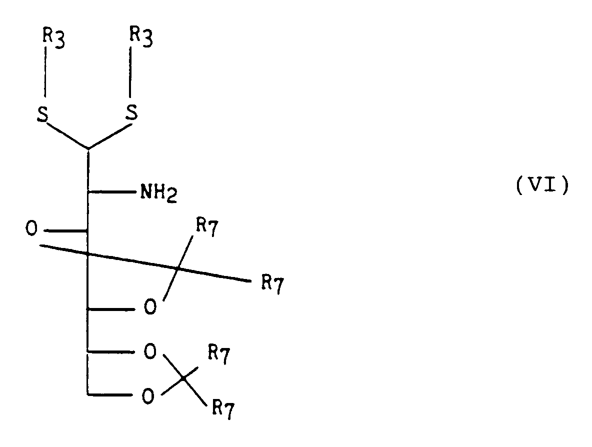 wherein R₃ and R₇ are as defined for formula I according to claim 1,
which is condensed with an aldehyde of formula VII: 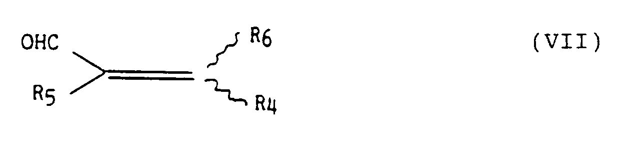 wherein R₄, R₅, R₆ and R₇ are as defined for formula I according to claim 1,
to obtain a Schiff's base of formula VIII: 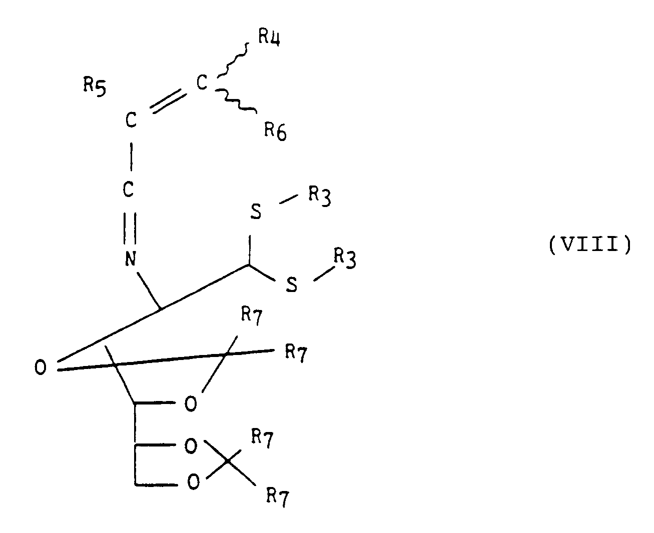 wherein R₃, R₄, R₅, R₆ and R₇ are as defined for formula I according to claim 1,
which is then cyclised in the presence of triethylamine with an acid chloride of formula IX: 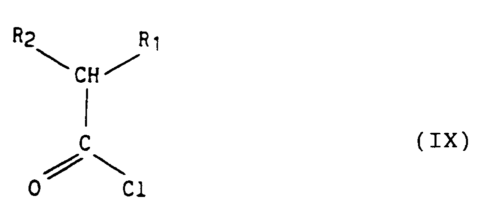 wherein R₁ and R₂ are as defined for formula I according to claim 1,
to obtain a mixture of monobactams of the general formulae I_{A} and I_{B} which is then separated by crystallisation and/or by preparative chromatography.

2. Use of compounds I_{A} and I_{B} according to claim 1 as intermediates for the preparation of the monobactams X_{A} and X_{B}: 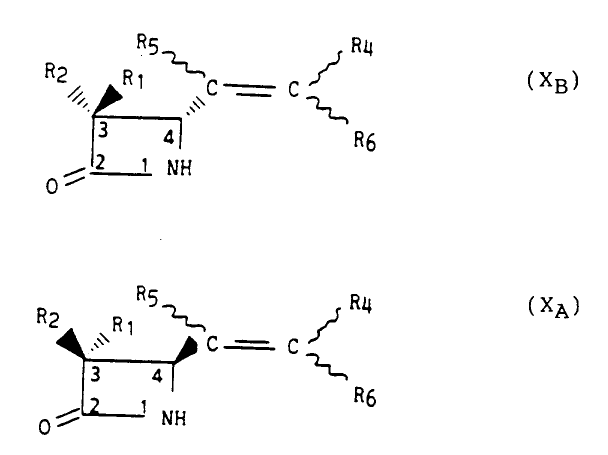 wherein R₁, R₂, R₄, R₅ and R₆ are as defined for formula I according to claim 1.

3. Use of the compounds of formulae I_{A} and I_{B} in the preparation of compounds X_{A} and X_{B} according to claim 2, characterised in that the compounds of formulae I_{A} and I_{B} are subjected to the action of n-butyllithium or another equivalent chemical reagent to obtain the compounds of formulae X_{A} and X_{B}.

4. Process for the preparation of compounds X_{A} and X_{B} according to claims 2 and 3, characterised in that the compounds of formulae I_{A} and I_{B} are subjected, in solution in tetrahydrofuran, or in another equivalent organic solvent, at a temperature of from -20°C to -80°C, to the action of n-butyllithium,
and then the reaction medium is neutralised with a mineral acid to obtain the compounds of formulae X_{A} and X_{B}.

## Claims (Claims for the following Contracting State(s): GR)

1. Compounds of formula I, constituted by the totality of formulae I_{A} and I_{B}: 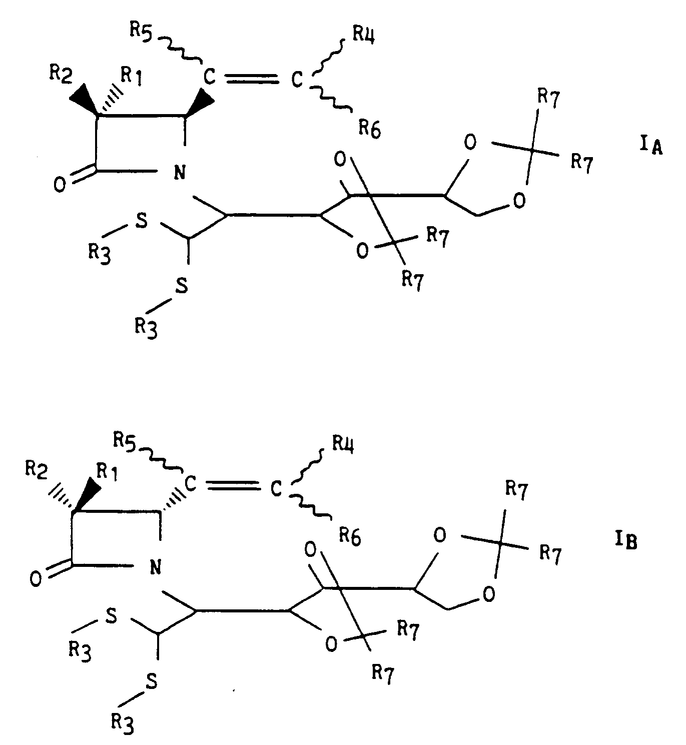 wherein:
R₁ represents a hydrogen atom, a halogen atom, an alkyl radical having from 1 to 6 carbon atoms, a trifluoromethyl radical, an alkoxy radical having from 1 to 6 carbon atoms, a phenyl or phenoxy radical or a benzyl radical (each of which is optionally substituted on the benzene ring by one or more halogen atoms, alkoxy radicals having from 1 to 6 carbon atoms or alkyl radicals having from 1 to 6 carbon atoms), a benzyloxy radical, a cyano radical, an alkoxycarbonyl radical having from 2 to 7 carbon atoms, a carboxy radical, or an alkylamino radical having from 1 to 6 carbon atoms (with the proviso, however, that in that case R₂ never simultaneously represents a hydrogen atom),
R₂, which differs from R₁, represents a hydrogen atom, a halogen atom, an alkyl radical having from 1 to 6 carbon atoms, a trifluoromethyl radical, an alkoxy radical having from 1 to 6 carbon atoms, a phenyl, phenoxy or benzyl radical (each of which is optionally substituted on the benzene ring by one or more halogen atoms, alkoxy radicals having from 1 to 6 carbon atoms or alkyl radicals having from 1 to 6 carbon atoms), a benzyloxy radical, a cyano radical, an alkoxycarbonyl radical having from 2 to 7 carbon atoms, a carboxy radical, or an alkylamino radical having from 1 to 6 carbon atoms (with the proviso, however, that in that case R₁ never simultaneously represents a hydrogen atom),
each R₃ represents an alkyl radical having from 1 to 6 carbon atoms or the two R₃ form together and with the atoms of the dithioacetal function to which they are attached a ring having from 5 to 7 ring members,
R₄ represents a phenyl radical (optionally substituted by one or more halogen atoms, by one or more alkyl radicals having from 1 to 6 carbon atoms, by an alkoxy radical having from 1 to 6 carbon atoms), a naphthyl radical or a furyl radical,
each of R₅ and R₆, which may be identical or different, represents a hydrogen atom, an alkyl radical having from 1 to 7 carbon atoms, or an aryl radical (optionally substituted by one or more halogen atoms, alkoxy radicals having from 1 to 6 carbon atoms or alkyl radicals having from 1 to 6 carbon atoms),
and
each R₇ represents a methyl radical or the two R₇ form together and with the carbon atom to which they are attached a cyclohexane ring or a cyclopentane ring.

2. Process for the preparation of the compounds of formulae I_{A} and I_{B} according to claim 1,
characterised in that D-glucosamine, a compound of formula II: 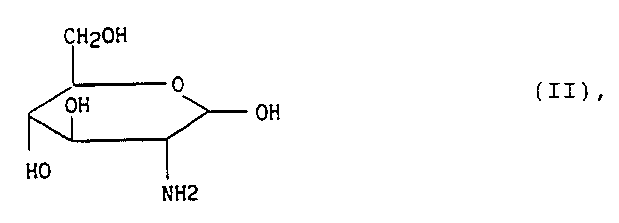 is condensed
. either with an equimolar amount of a compound of formula III_{A}:
HS(CH₂)ₙSH III_{A}
wherein n is 2 to 4,
. or with at least double the amount of a compound of formula III_{B}:
HSR'₃ III_{B}
wherein R'₃ represents an alkyl radical having from 1 to 6 carbon atoms,
in the presence of concentrated hydrochloric acid to obtain a D-glucosamine compound of formula IV: 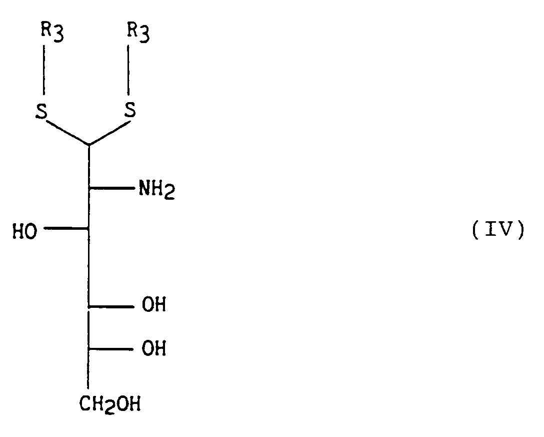 wherein R₃ is as defined for formula I according to claim 1,
with which is reacted a compound of formula V: 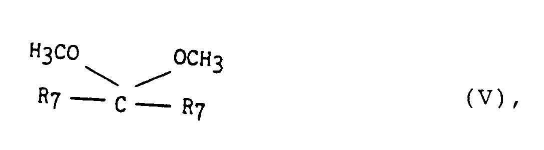 wherein R₇ is as defined for formula I according to claim 1, in the presence of paratoluenesulphonic acid,
to obtain a compound of formula VI: 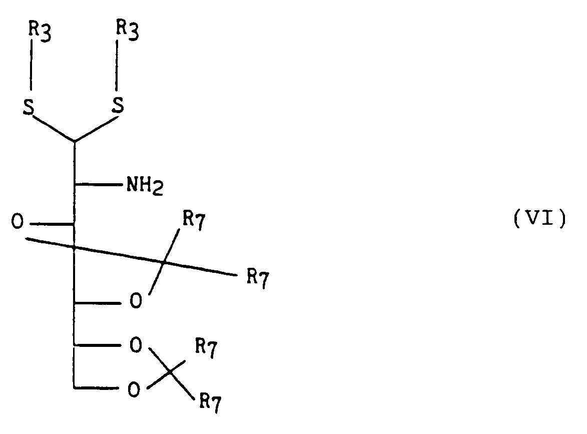 wherein R₃ and R₇ are as defined for formula I according to claim 1,
which is condensed with an aldehyde of formula VII: 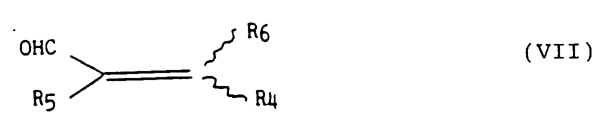 wherein R₄, R₅, R₆ and R₇ are as defined for formula I according to claim 1,
to obtain a Schiff's base of formula VIII: 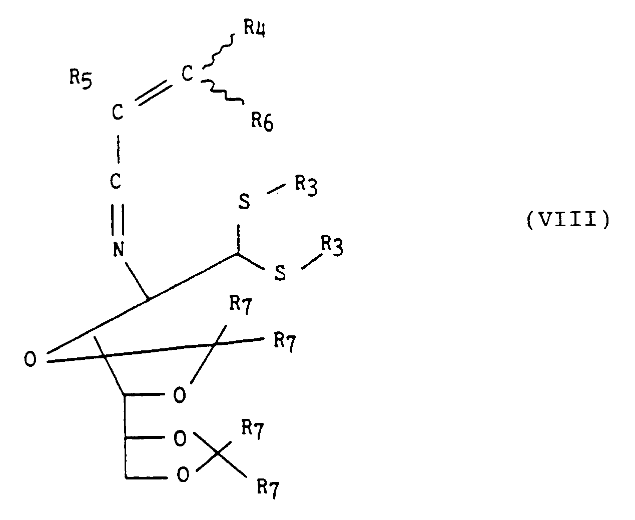 wherein R₃, R₄, R₅, R₆ and R₇ are as defined for formula I according to claim 1,
which is then cyclised in the presence of triethylamine with an acid chloride of formula IX: 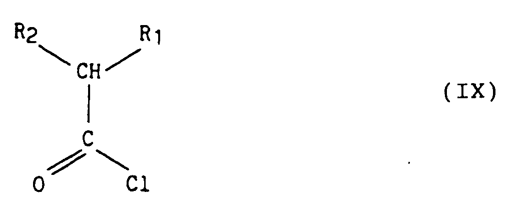 wherein R₁ and R₂ are as defined for formula I according to claim 1,
to obtain a mixture of monobactams of the general formulae I_{A} and I_{B} which is then separated by crystallisation and/or by preparative chromatography.

3. Use of compounds I_{A} and I_{B} according to claim 1 as intermediates for the preparation of the monobactams X_{A} and X_{B}: 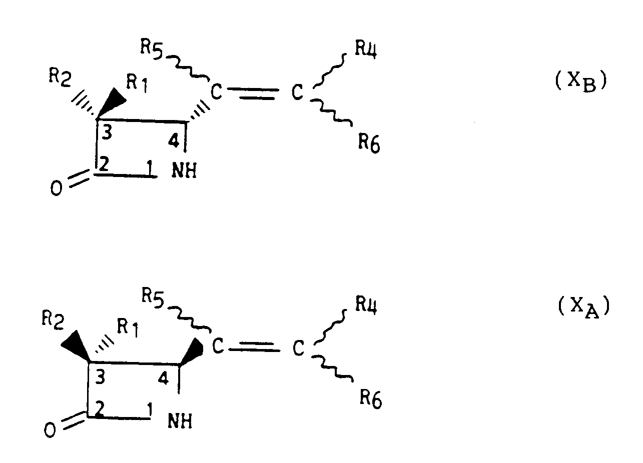 wherein R₁, R₂, R₄, R₅ and R₆ are as defined for formula I according to claim 1.

4. Use of the compounds of formulae I_{A} and I_{B} in the preparation of compounds X_{A} and X_{B} according to claim 3, characterised in that the compounds of formulae I_{A} and I_{B} are subjected to the action of n-butyllithium or another equivalent chemical reagent to obtain the compounds of formulae X_{A} and X_{B}.

5. Process for the preparation of compounds X_{A} and X_{B} according to claims 3 and 4, characterised in that the compounds of formulae I_{A} and I_{B} are subjected, in solution in tetrahydrofuran, or in another equivalent organic solvent, at a temperature of from -20°C to -80°C, to the action of n-butyllithium,
and then the reaction medium is neutralised with a mineral acid to obtain the compounds of formulae X_{A} and X_{B}.

## Patentansprüche (Patentansprüche für folgende(n) Vertragsstaat(en): AT, BE, CH, DE, DK, FR, GB, IT, LI, LU, NL, SE)

1. Verbindungen der Formel I, umfaßt durch die Gesamtheit der Formeln I_{A} und I_{B}: 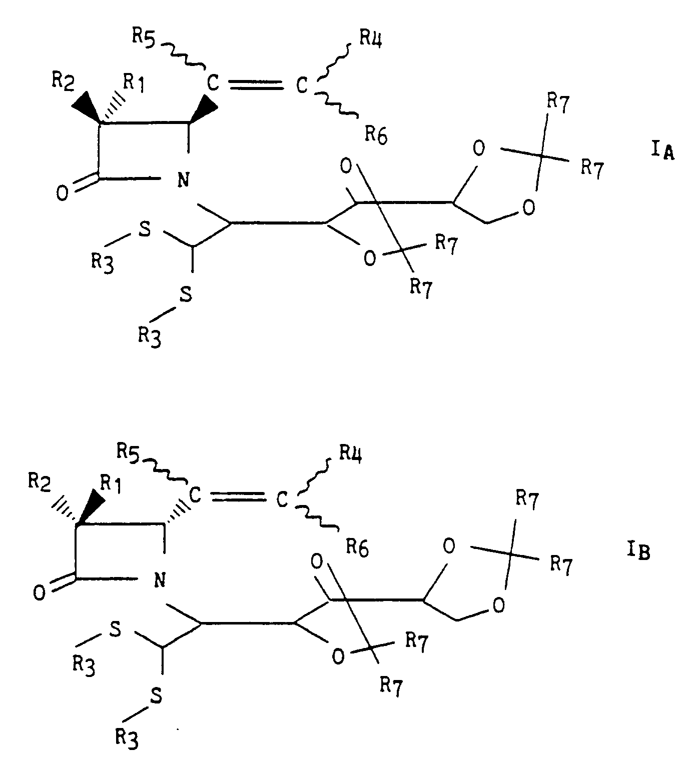 worin:
R₁ ein Wasserstoffatom, ein Halogenatom, eine Alkylgruppe mit 1 bis 6 Kohlenstoffatomen, eine Trifluormethylgruppe, eine Alkoxygruppe mit 1 bis 6 Kohlenstoffatomen, eine Phenyl-, Phenoxy- oder Benzyl-gruppe (die gegebenenfalls am Benzolring durch ein oder mehrere Halogenatome, Alkoxygruppen mit 1 bis 6 Kohlenstoffatomen oder Alkylgruppen mit 1 bis 6 Kohlenstoffatomen substituiert sein können), eine Benzyloxygruppe, eine Cyanogruppe, eine Alkoxycarbonylgruppe mit 2 bis 7 Kohlenstoffatomen, eine Carboxygruppe oder eine Alkylaminogruppe mit 1 bis 6 Kohlenstoffatomen (mit der Maßgabe, daß in diesem Fall R₂ nicht gleichzeitig ein Wasserstoffatom darstellt),
R₂, welches von R₁ verschieden ist, ein Wasserstoffatom, ein Halogenatom, eine Alkylgruppe mit 1 bis 6 Kohlenstoffatomen, eine Trifluormethylgruppe, eine Alkoxygruppe mit 1 bis 6 Kohlenstoffatomen, eine Phenyl-, Phenoxy- oder Benzylgruppe (die gegebenenfalls am Benzolring durch ein oder mehrere Halogenatome, Alkoxygruppen mit 1 bis 6 Kohlenstoffatomen oder Alkylgruppen mit 1 bis 6 Kohlenstoffatomen substituiert sein können), eine Benzyloxygruppe, eine Cyanogruppe, eine Alkoxycarbonylgruppe mit 2 bis 7 Kohlenstoffatomen, eine Carboxygruppe oder eine Alkylaminogruppe mit 1 bis 6 Kohlenstoffatomen (mit der Maßgabe, daß in diesem Fall R₁ nicht gleichzeitig ein Wasserstoffatom darstellt).
R₃ jeweils eine Alkylgruppe mit 1 bis 6 Kohlenstoffatomen oder die beiden Gruppen R₃ gemeinsam mit den Atomen der Dithioacetalgruppe, an die sie gebunden sind, einen Ring mit 5 bis 7 Kettengliedern.
R₄ eine Phenylgruppe (die gegebenenfalls durch ein oder mehrere Halogenatome, ein oder mehrere Alkylgruppen mit 1 bis 6 Kohlenstoffatomen oder eine Alkoxygruppe mit 1 bis 6 Kohlenstoffatomen substituiert ist), eine Naphthylgruppe oder eine Furylgruppe.
R₅ und R₆, die gleichartig oder verschieden sein können, jeweils ein Wasserstoffatom, eine Alkylgruppe mit 1 bis 7 Kohlenstoffatomen oder eine Arylgruppe (die gegebenenfalls durch ein oder mehrere Halogenatome, Alkoxygruppen mit 1 bis 6 Kohlenstoffatomen oder Alkylgruppen mit 1 bis 6 Kohlenstoffatomen substituiert ist)
und
R₇ jeweils eine Methylgruppe oder die beiden Gruppen R₇ zusammen mit dem Kohlenstoffatom, an das sie gebunden sind, einen Cyclohexanring oder einen Cyclopentanring
bedeuten.

2. Verfahren zur Herstellung der Verbindungen der Formeln I_{A} und I_{B} nach Anspruch 1, **dadurch gekennzeichnet**, daß man D-Glucosamin der Formel II: 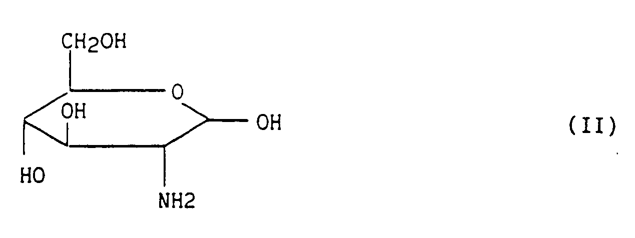
. entweder mit einer äquimolaren Menge einer Verbindung der Formel III_{A}:
HS(CH₂)ₙSH III_{A}
in der n den Wert 2 bis 4 besitzt,
. oder mit einer mindestens doppelten Menge einer Verbindung der Formel III_{B}:
HSR'₃ III_{B}
in der R'₃ eine Alkylgruppe mit 1 bis 6 Kohlenstoffatomen darstellt,
in Gegenwart von konzentrierter Chlorwasserstoffsäure kondensiert zur Bildung eines D-Glucosaminderivats der Formel IV: 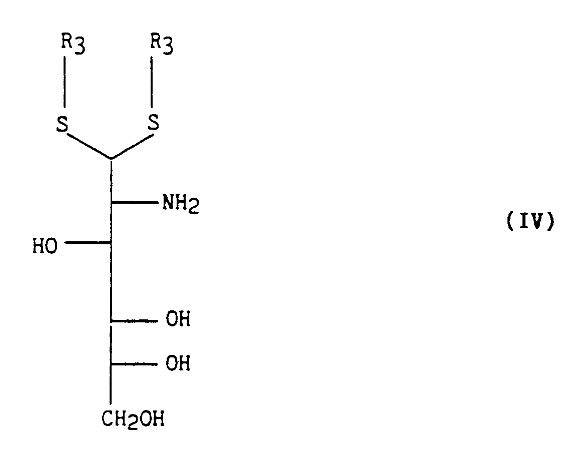 in der R₃ die bezüglich der Formel I In Anspruch 1 angegebenen Bedeutungen besitzt,
welches man mit einer Verbindung der Formel V: 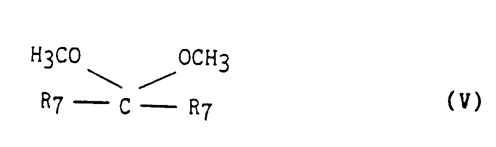 in der R₇ die bezüglich der Formel I in Anspruch 1 angegebenen Bedeutungen besitzt, in Gegenwart von p-Toluolsulfonsäure umsetzt,
zur Bildung eines Derivats der Formel VI: 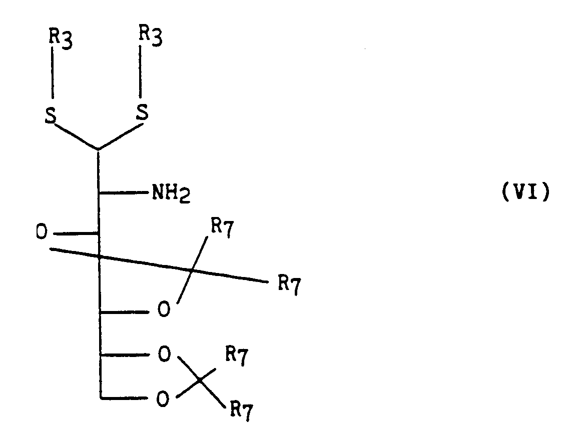 in der R₃ und R₇ die bezüglich der Formel I in Anspruch 1 angegebenen Bedeutungen besitzen,
welches man mit einem Aldehyd der Formel VII: 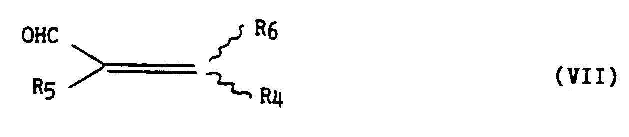 in der R₄, R₅, R₆ und R₇ die bezüglich der Formel I in Anspruch 1 angegebenen Bedeutungen besitzen, kondensiert,
zur Bildung einer Schiffschen Base der Formel VIII: 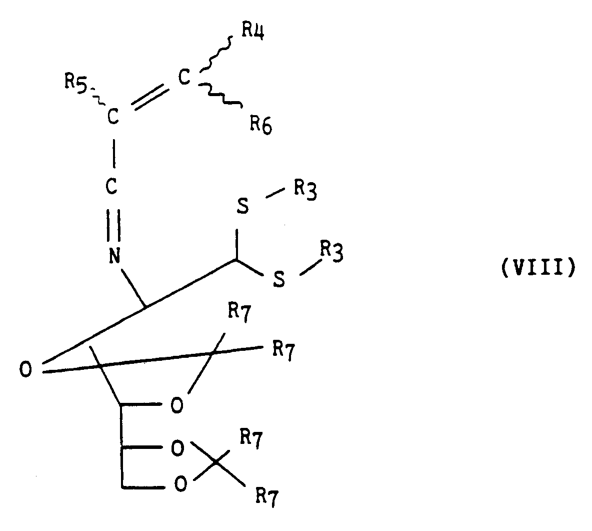 in der R₃, R₄, R₅, R₆ und R₇ die bezüglich der Formel I in Anspruch 1 angegebenen Bedeutungen besitzen,
welche man anschließend in Gegenwart von Triethylamin mit einem Säurechlorid der Formel IX: 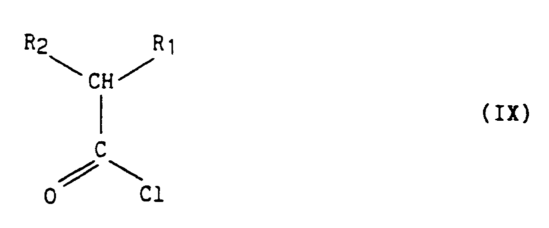 in der R₁ und R₂ die bezüglich der Formel I in Anspruch 1 angegebenen Bedeutungen besitzen, cyclisiert,
so daß man eine Mischung von Monobactamen der allgemeinen Formel I_{A} und I_{B}, die man anschließend durch Kristallisation und/oder durch präparative Chromatographie trennt.

3. Verwendung der Verbindungen I_{A} und I_{B} nach Anspruch 1 als Zwischenprodukte zur Herstellung von Monobactamen X_{A} und X_{B}: 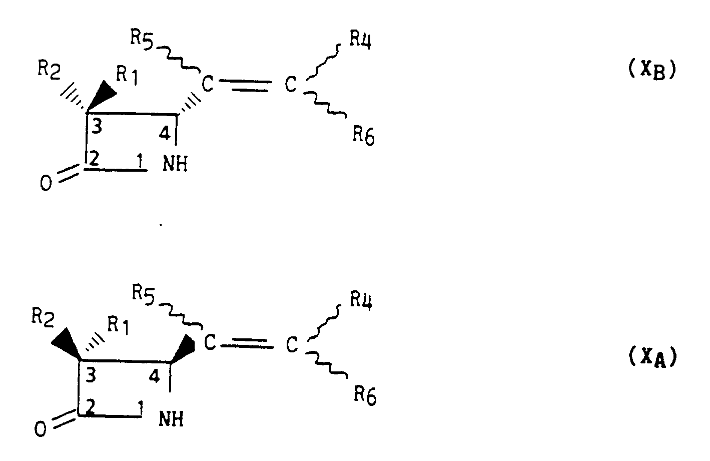 worin R₁, R₂, R₄, R₅ und R₆ die bezüglich der Formel I in Anspruch 1 angegebenen Bedeutungen besitzen.

4. Verwendung der Verbindungen der Formeln I_{A} und I_{B} zur Herstellung der Verbindungen X_{A} und X_{B} nach Anspruch 3, **dadurch gekennzeichnet**, daß man die Verbindungen der Formeln I_{A} und I_{B} der Einwirkung von n-Butyllithium oder eines anderen chemisch äquivalenten Reagens unterwirft zur Bildung der Verbindungen der Formeln X_{A} und X_{B}.

5. Verfahren zur Herstellung der Verbindungen X_{A} und X_{B} nach den Ansprüchen 3 und 4, **dadurch gekennzeichnet**, daß man die Verbindungen der Formeln I_{A} und I_{B} in Lösung in Tetrahydrofuran oder in einem anderen äquivalenten organischen Lösungsmittel bei einer Temperatur zwischen -20°C und -80°C der Einwirkung von n-Butyllithium unterwirft
und dann das Reaktionsmedium mit Hilfe einer anorganischen Säure neutralisiert unter Erhalt der Verbindungen der Formeln X_{A} und X_{B}.

## Patentansprüche (Patentansprüche für folgende(n) Vertragsstaat(en): ES)

1. Verfahren zur Herstellung der Verbindungen der Formel I, umfaßt durch die Gesamtheit der Formeln I_{A} und I_{B}: 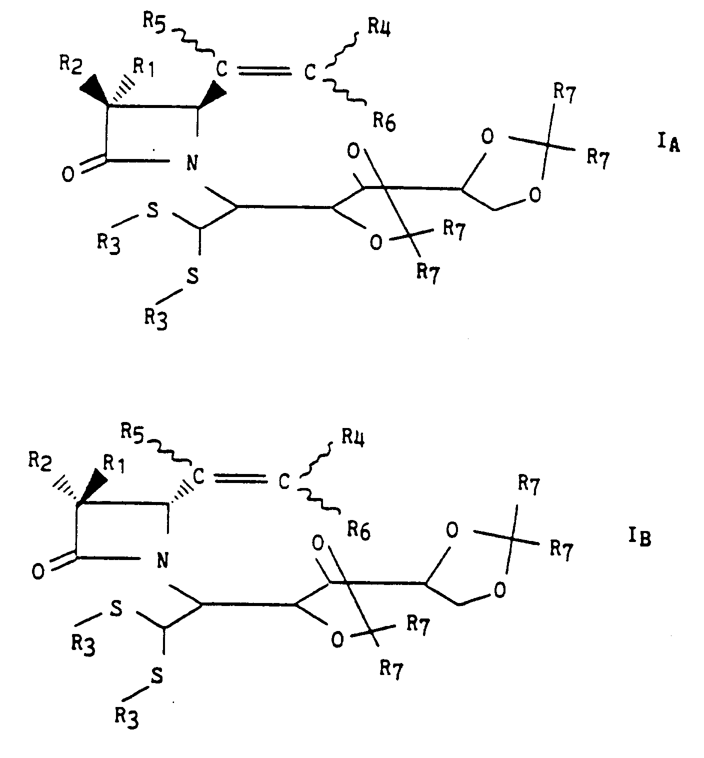 worin:
R₁ ein Wasserstoffatom, ein Halogenatom, eine Alkylgruppe mit 1 bis 6 Kohlenstoffatomen, eine Trifluormethylgruppe, eine Alkoxygruppe mit 1 bis 6 Kohlenstoffatomen, eine Phenyl-, Phenoxy- oder Benzyl-gruppe (die gegebenenfalls am Benzolring durch ein oder mehrere Halogenatome, Alkoxygruppen mit 1 bis 6 Kohlenstoffatomen oder Alkylgruppen mit 1 bis 6 Kohlenstoffatomen substituiert sein können), eine Benzyloxygruppe, eine Cyanogruppe, eine Alkoxycarbonylgruppe mit 2 bis 7 Kohlenstoffatomen, eine Carboxygruppe oder eine Alkylaminogruppe mit 1 bis 6 Kohlenstoffatomen (mit der Maßgabe, daß in diesem Fall R₂ nicht gleichzeitig ein Wasserstoffatom darstellt),
R₂, welches von R₁ verschieden ist, ein Wasserstoffatom, ein Halogenatom, eine Alkylgruppe mit 1 bis 6 Kohlenstoffatomen, eine Trifluormethylgruppe, eine Alkoxygruppe mit 1 bis 6 Kohlenstoffatomen, eine Phenyl-, Phenoxy- oder Benzylgruppe (die gegebenenfalls am Benzolring durch ein oder mehrere Halogenatome, Alkoxygruppen mit 1 bis 6 Kohlenstoffatomen oder Alkylgruppen mit 1 bis 6 Kohlenstoffatomen substituiert sein können), eine Benzyloxygruppe, eine Cyanogruppe, eine Alkoxycarbonylgruppe mit 2 bis 7 Kohlenstoffatomen, eine Carboxygruppe oder eine Alkylaminogruppe mit 1 bis 6 Kohlenstoffatomen (mit der Maßgabe, daß in diesem Fall R₁ nicht gleichzeitig ein Wasserstoffatom darstellt),
R₃ jeweils eine Alkylgruppe mit 1 bis 6 Kohlenstoffatomen oder die beiden Gruppen R₃ gemeinsam mit den Atomen der Dithioacetalgruppe, an die sie gebunden sind, einen Ring mit 5 bis 7 Kettengliedern,
R₄ eine Phenylgruppe (die gegebenenfalls durch ein oder mehrere Halogenatome, ein oder mehrere Alkylgruppen mit 1 bis 6 Kohlenstoffatomen oder eine Alkoxygruppe mit 1 bis 6 Kohlenstoffatomen substituiert ist), eine Naphthylgruppe oder eine Furylgruppe,
R₅ und R₆, die gleichartig oder verschieden sein können, jeweils ein Wasserstoffatom, eine Alkylgruppe mit 1 bis 7 Kohlenstoffatomen oder eine Arylgruppe (die gegebenenfalls durch ein oder mehrere Halogenatome, Alkoxygruppen mit 1 bis 6 Kohlenstoffatomen oder Alkylgruppen mit 1 bis 6 Kohlenstoffatomen substituiert ist)
und
R₇ jeweils eine Methylgruppe oder die beiden Gruppen R₇ zusammen mit dem Kohlenstoffatom, an das sie gebunden sind, einen Cyclohexanring oder einen Cyclopentanring
bedeuten, **dadurch gekennzeichnet**, daß man D-Glucosamin der Formel II: 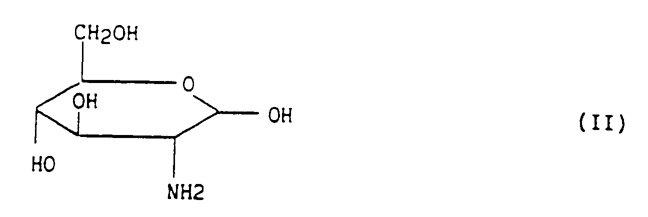
. entweder mit einer äquimolaren Menge einer Verbindung der Formel III_{A}:
HS(CH₂)ₙSH III_{A}
in der n den Wert 2 bis 4 besitzt,
. oder mit einer mindestens doppelten Menge einer Verbindung der Formel III_{B}:
HSR'₃ III_{B}
in der R'₃ eine Alkylgruppe mit 1 bis 6 Kohlenstoffatomen darstellt,
in Gegenwart von konzentrierter Chlorwasserstoffsäure kondensiert zur Bildung eines D-Glucosaminderivats der Formel IV: 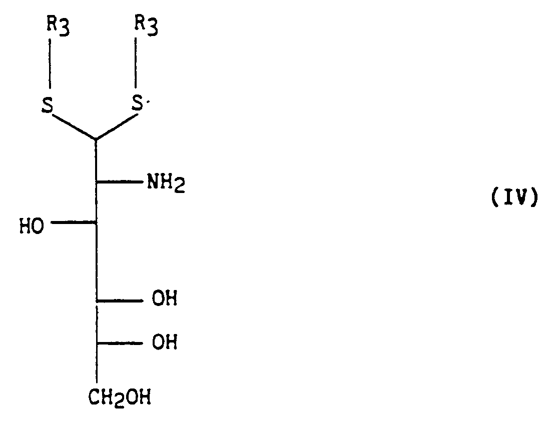 in der R₃ die bezüglich der Formel I angegebenen Bedeutungen besitzt, welches man mit einer Verbindung der Formel V: 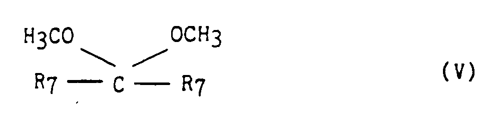 in der R₇ die bezüglich der Formel I angegebenen Bedeutungen besitzt, in Gegenwart von p-Toluolsulfonsäure umsetzt,
zur Bildung eines Derivats der Formel VI: 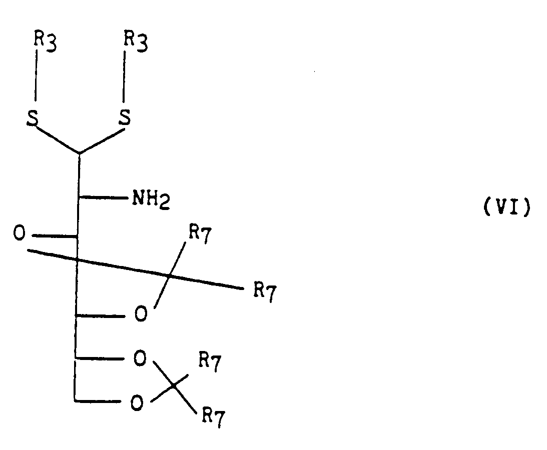 in der R₃ und R₇ die bezüglich der Formel I angegebenen Bedeutungen besitzen,
welches man mit einem Aldehyd der Formel VII: 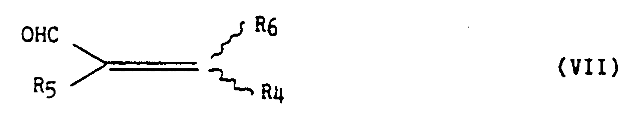 in der R₄, R₅, R₆ und R₇ die bezüglich der Formel I angegebenen Bedeutungen besitzen, kondensiert,
zur Bildung einer Schiffschen Base der Formel VIII: 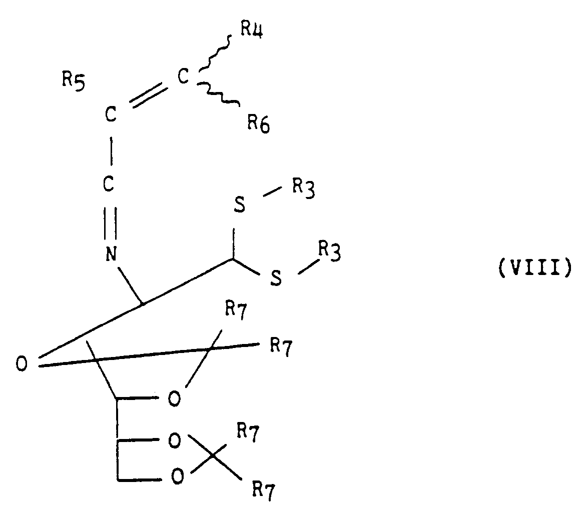 in der R₃, R₄, R₅, R₆ und R₇ die bezüglich der Formel I angegebenen Bedeutungen besitzen,
welche man anschließend in Gegenwart von Triethylamin mit einem Säurechlorid der Formel IX: 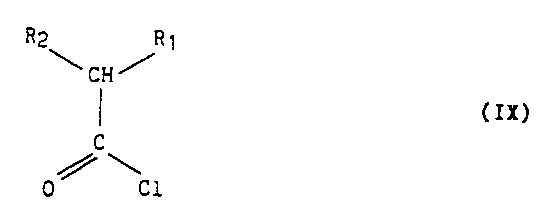 in der R₁ und R₂ die bezüglich der Formel I angegebenen Bedeutungen besitzen, cyclisiert,
so daß man eine Mischung von Monobactamen der allgemeinen Formel I_{A} und I_{B}, die man anschließend durch Kristallisation und/oder durch präparative Chromatographie trennt.

2. Verwendung der Verbindungen I_{A} und I_{B} nach Anspruch 1 als Zwischenprodukte zur Herstellung von Monobactamen X_{A} und X_{B}: 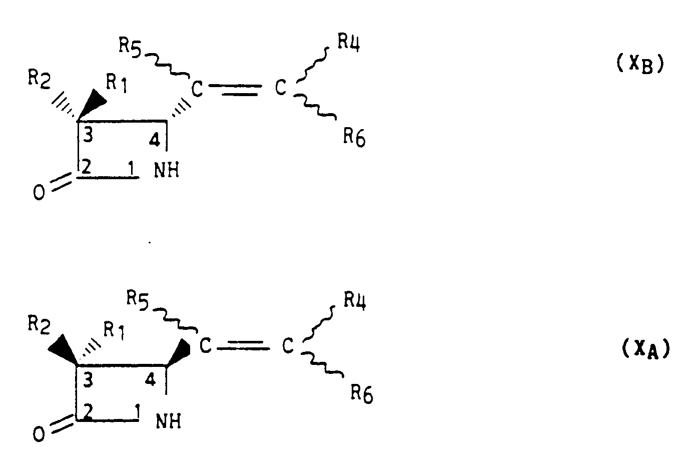 worin R₁, R₂, R₄, R₅ und R₆ die bezüglich der Formel I in Anspruch 1 angegebenen Bedeutungen besitzen.

3. Verwendung der Verbindungen der Formeln I_{A} und I_{B} zur Herstellung der Verbindungen X_{A} und X_{B} nach Anspruch 2, **dadurch gekennzeichnet**, daß man die Verbindungen der Formeln I_{A} und I_{B} der Einwirkung von n-Butyllithium oder eines anderen chemisch äquivalenten Reagens unterwirft zur Bildung der Verbindungen der Formeln X_{A} und X_{B}.

4. Verfahren zur Herstellung der Verbindungen X_{A} und X_{B} nach den Ansprüchen 3 und 4, **dadurch gekennzeichnet**, daß man die Verbindungen der Formeln I_{A} und I_{B} in Lösung in Tetrahydrofuran oder in einem anderen äquivalenten organischen Lösungsmittel bei einer Temperatur zwischen -20°C und -80°C der Einwirkung von n-Butyllithium unterwirft
und dann das Reaktionsmedium mit Hilfe einer anorganischen Säure neutralisiert unter Erhalt der Verbindungen der Formeln X_{A} und X_{B}.

## Patentansprüche (Patentansprüche für folgende(n) Vertragsstaat(en): GR)

1. Verbindungen der Formel I, umfaßt durch die Gesamtheit der Formeln I_{A} und I_{B}: 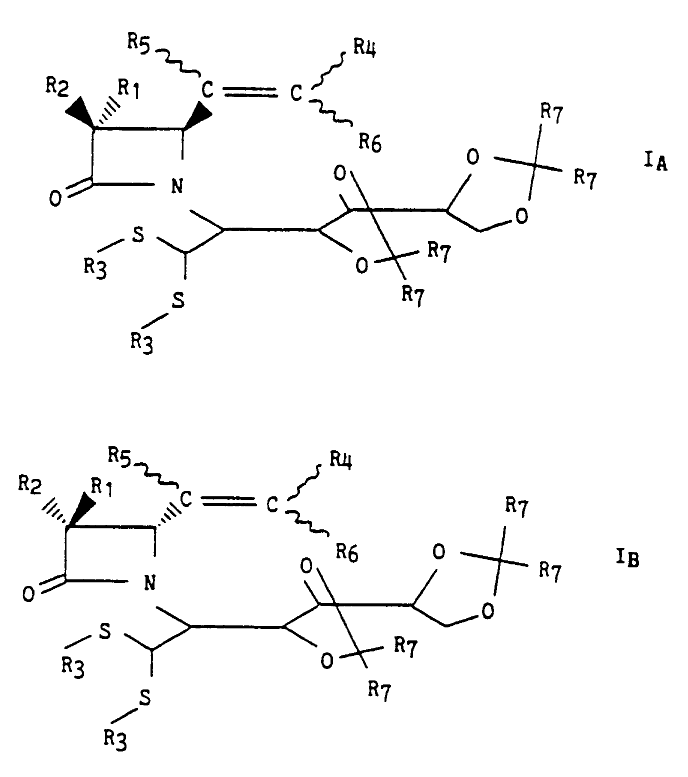 worin:
R₁ ein Wasserstoffatom, ein Halogenatom, eine Alkylgruppe mit 1 bis 6 Kohlenstoffatomen, eine Trifluormethylgruppe, eine Alkoxygruppe mit 1 bis 6 Kohlenstoffatomen, eine Phenyl-, Phenoxy- oder Benzyl-gruppe (die gegebenenfalls am Benzolring durch ein oder mehrere Halogenatome, Alkoxygruppen mit 1 bis 6 Kohlenstoffatomen oder Alkylgruppen mit 1 bis 6 Kohlenstoffatomen substituiert sein können), eine Benzyloxygruppe, eine Cyanogruppe, eine Alkoxycarbonylgruppe mit 2 bis 7 Kohlenstoffatomen, eine Carboxygruppe oder eine Alkylaminogruppe mit 1 bis 6 Kohlenstoffatomen (mit der Maßgabe, daß in diesem Fall R₂ nicht gleichzeitig ein Wasserstoffatom darstellt),
R₂, welches von R₁ verschieden ist, ein Wasserstoffatom, ein Halogenatom, eine Alkylgruppe mit 1 bis 6 Kohlenstoffatomen, eine Trifluormethylgruppe, eine Alkoxygruppe mit 1 bis 6 Kohlenstoffatomen, eine Phenyl-, Phenoxy- oder Benzylgruppe (die gegebenenfalls am Benzolring durch ein oder mehrere Halogenatome, Alkoxygruppen mit 1 bis 6 Kohlenstoffatomen oder Alkylgruppen mit 1 bis 6 Kohlenstoffatomen substituiert sein können), eine Benzyloxygruppe, eine Cyanogruppe, eine Alkoxycarbonylgruppe mit 2 bis 7 Kohlenstoffatomen, eine Carboxygruppe oder eine Alkylaminogruppe mit 1 bis 6 Kohlenstoffatomen (mit der Maßgabe, daß in diesem Fall R₁ nicht gleichzeitig ein Wasserstoffatom darstellt),
R₃ jeweils eine Alkylgruppe mit 1 bis 6 Kohlenstoffatomen oder die beiden Gruppen R₃ gemeinsam mit den Atomen der Dithioacetalgruppe, an die sie gebunden sind, einen Ring mit 5 bis 7 Kettengliedern,
R₄ eine Phenylgruppe (die gegebenenfalls durch ein oder mehrere Halogenatome, ein oder mehrere Alkylgruppen mit 1 bis 6 Kohlenstoffatomen oder eine Alkoxygruppe mit 1 bis 6 Kohlenstoffatomen substituiert ist), eine Naphthylgruppe oder eine Furylgruppe,
R₅ und R₆, die gleichartig oder verschieden sein können, jeweils ein Wasserstoffatom, eine Alkylgruppe mit 1 bis 7 Kohlenstoffatomen oder eine Arylgruppe (die gegebenenfalls durch ein oder mehrere Halogenatome, Alkoxygruppen mit 1 bis 6 Kohlenstoffatomen oder Alkylgruppen mit 1 bis 6 Kohlenstoffatomen substituiert ist)
und
R₇ jeweils eine Methylgruppe oder die beiden Gruppen R₇ zusammen mit dem Kohlenstoffatom, an das sie gebunden sind, einen Cyclohexanring oder einen Cyclopentanring
bedeuten.

2. Verfahren zur Herstellung der Verbindungen der Formeln I_{A} und I_{B} nach Anspruch 1, **dadurch gekennzeichnet**, daß man D-Glucosamin der Formel II: 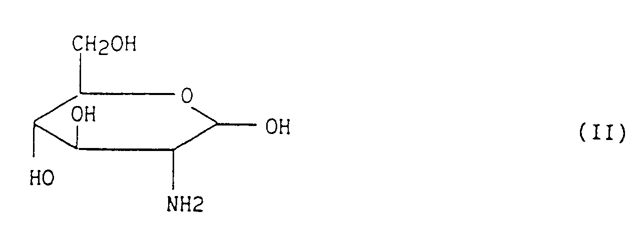
. entweder mit einer äquimolaren Menge einer Verbindung der Formel III_{A}:
HS(CH₂)ₙSH III_{A}
in der n den Wert 2 bis 4 besitzt,
. oder mit einer mindestens doppelten Menge einer Verbindung der Formel III_{B}:
HSR'₃ III_{B}
in der R'₃ eine Alkylgruppe mit 1 bis 6 Kohlenstoffatomen darstellt,
in Gegenwart von konzentrierter Chlorwasserstoffsäure kondensiert zur Bildung eines D-Glucosaminderivats der Formel IV: 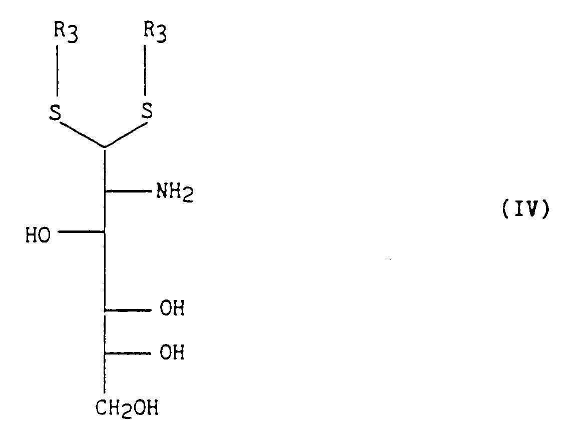 in der R₃ die bezüglich der Formel I in Anspruch 1 angegebenen Bedeutungen besitzt,
welches man mit einer Verbindung der Formel V: 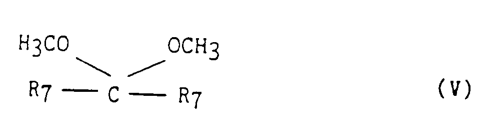 in der R₇ die bezüglich der Formel I In Anspruch 1 angegebenen Bedeutungen besitzt, in Gegenwart von p-Toluolsulfonsäure umsetzt,
zur Bildung eines Derivats der Formel VI: 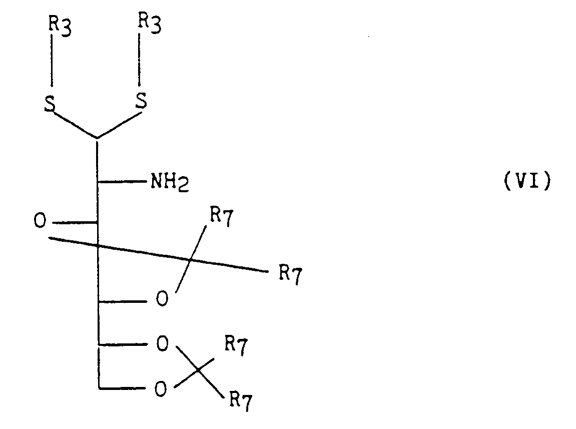 in der R₃ und R₇ die bezüglich der Formel I in Anspruch 1 angegebenen Bedeutungen besitzen,
welches man mit einem Aldehyd der Formel VII: 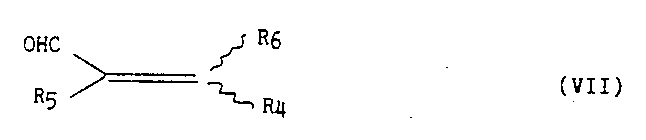 in der R₄, R₅, R₆ und R₇ die bezüglich der Formel I in Anspruch 1 angegebenen Bedeutungen besitzen, kondensiert,
zur Bildung einer Schiffschen Base der Formel VIII: 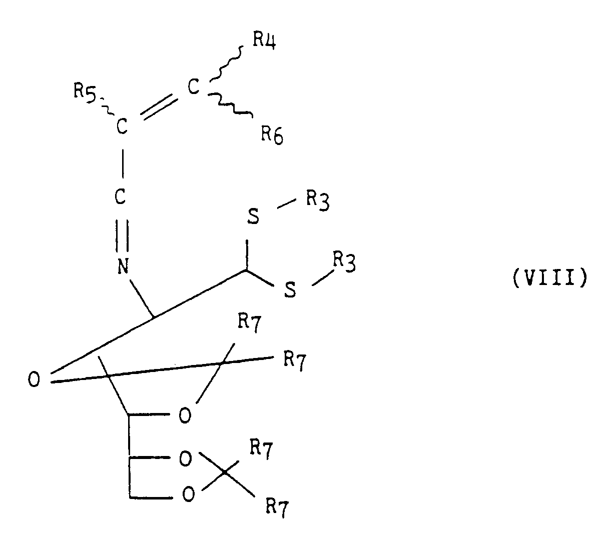 in der R₃, R₄, R₅, R₆ und R₇ die bezüglich der Formel I in Anspruch 1 angegebenen Bedeutungen besitzen,
welche man anschließend in Gegenwart von Triethylamin mit einem Säurechlorid der Formel IX: 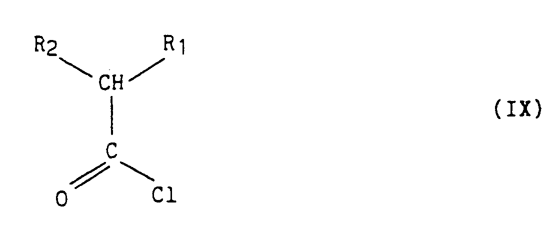 in der R₁ und R₂ die bezüglich der Formel I in Anspruch 1 angegebenen Bedeutungen besitzen, cyclisiert,
so daß man eine Mischung von Monobactamen der allgemeinen Formel I_{A} und I_{B}, die man anschließend durch Kristallisation und/oder durch präparative Chromatographie trennt.

3. Verwendung der Verbindungen I_{A} und I_{B} nach Anspruch 1 als Zwischenprodukte zur Herstellung von Monobactamen X_{A} und X_{B}: 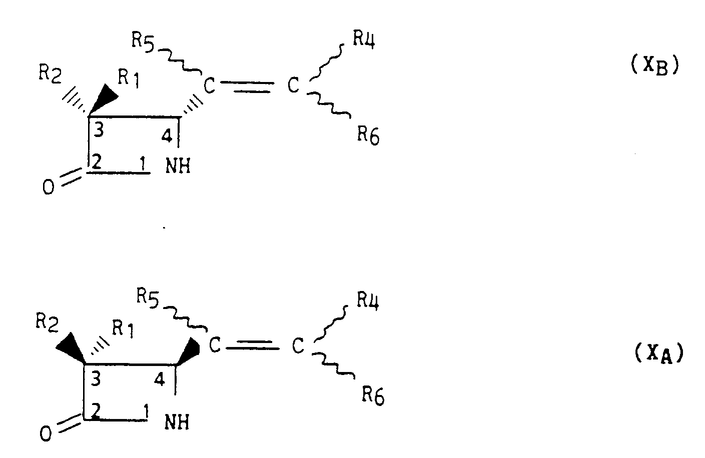 worin R₁, R₂, R₄, R₅ und R₆ die bezüglich der Formel I in Anspruch 1 angegebenen Bedeutungen besitzen.

4. Verwendung der Verbindungen der Formeln I_{A} und I_{B} zur Herstellung der Verbindungen X_{A} und X_{B} nach Anspruch 3, **dadurch gekennzeichnet**, daß man die Verbindungen der Formeln I_{A} und I_{B} der Einwirkung von n-Butyllithium oder eines anderen chemisch äquivalenten Reagens unterwirft zur Bildung der Verbindungen der Formeln X_{A} und X_{B}.

5. Verfahren zur Herstellung der Verbindungen X_{A} und X_{B} nach den Ansprüchen 3 und 4, **dadurch gekennzeichnet**, daß man die Verbindungen der Formeln I_{A} und I_{B} in Lösung in Tetrahydrofuran oder in einem anderen äquivalenten organischen Lösungsmittel bei einer Temperatur zwischen -20°C und -80°C der Einwirkung von n-Butyllithium unterwirft
und dann das Reaktionsmedium mit Hilfe einer anorganischen Säure neutralisiert unter Erhalt der Verbindungen der Formeln X_{A} und X_{B}.
